# EUROPÄISCHE PATENTSCHRIFT

(11) **EP 2 096 481 B1**
(45) Veröffentlichungstag und Bekanntmachung des Hinweises auf die Patenterteilung: **02.04.2014**
(21) Anmeldenummer: 09153268.9
(22) Anmeldetag: 20.02.2009
(51) Int. Cl.: G02B 21/00, A61B 3/13, A61B 19/00, G02B 21/22, G02B 7/02

(54) **Vorsatzeinrichtung für eine optische Beobachtungseinrichtung**
Header device for an optical observation device
Dispositif de montage pour un dispositif d'observation optique

(30) Priorität: 28.02.2008 DE 102008011608
(43) Veröffentlichungstag der Anmeldung: 02.09.2009
(60) Teilanmeldung: 10157742.7
(73) Patentinhaber: Carl Zeiss Meditec AG, 07745 Jena (DE)
(72) Erfinder: Takanashi, Fumio, 73447 Oberkochen (DE); Seeßelberg, Markus, 73431 Aalen (DE); Müller, Andrè, 89551 Königsbronn-Zang (DE); Reimer, Peter, 73479 Ellwangen (DE)
(74) Vertreter: Müller & Schubert

(56) Entgegenhaltungen:
- EP-A- 1 447 698
- EP-A- 1 450 194
- WO-A-2004/019107
- DE-U1- 9 217 517
- DE-U1- 9 415 219
- DE-U1- 20 017 891
- DE-U1- 20 215 635
- US-A- 5 886 812

## Beschreibung

Die vorliegende Erfindung betrifft zunächst eine Vorsatzeinrichtung für ein Operationsmikroskop gemäß dem Oberbegriff von Patentanspruch 1.

Bei Operationsmikroskopen kann es sich insbesondere um Operationsmikroskope für die Ophthalmologie handeln. Solche Mikroskope weisen in der Regel unter anderem einen Mikroskopkörper mit verschiedenen optischen Elementen und einen Tubus auf. Weiterhin verfügen Mikroskope in der Regel über wenigstens ein Objektivelement. Nicht selten werden Vorsatzeinrichtungen im Umgebungsbereich des Objektivelements am Mikroskop befestigt.

Vorsatzeinrichtungen für Mikroskope sind bereits in verschiedenen Ausführungsformen bekannt. Sie dienen in der Regel dazu, zusätzliche Linsenelemente in einen Strahlengang des Mikroskops einzubringen. Ein Anwendungsbereich für solche Vorsatzeinrichtungen liegt auf dem Gebiet der indirekten Ophthalmoskopie.

Die Netzhaut beziehungsweise Bereiche des Glaskörpers eines Auges lassen sich mit Hilfe eines zusätzlichen optischen Bauelementes in Form wenigstens eines Linsenelements, beispielsweise eine hochbrechende einfache Linse oder Linsengruppe, das definiert vor dem Auge positioniert wird, abbilden. Die so gewonnene Abbildung des hinteren Augenabschnittes lässt sich bei Bedarf mit entsprechenden Beobachtungseinrichtungen, insbesondere mit einem stereoskopischen Operationsmikroskop, betrachten. Dabei ist die Lage / Position des Bildes abhängig von der verwendeten Ophthalmoskopierlupe, insbesondere hinsichtlich deren Brechkraft, von der Fehlsichtigkeit des zu betrachtenden Auges, etwa einer Kurz- oder Weitsichtigkeit, vom zu betrachtenden Bereich/Abschnitt des Auges, beispielsweise der Netzhaut, Bereiche des Glaskörpers im Abstand x über der Netzhaut oder dergleichen, von Anomalien des Auges, beispielsweisenatürlicher Glaskörper « flüssigkeits-/ölgefüllt « gasgefüllt; Phakie « Aphakie « Pseudophakie; und dergleichen, vom Abstand der Ophthalmoskopierlupe zum Patientenauge, und dergleichen.

Mittels der Vorsatzeinrichtung wird das optische Bauelement entweder direkt auf das zu betrachtende Auge aufgesetzt, beispielsweise in Form eines indirekten Kontaktglases, oder schwebend in einem bestimmten Abstand vor dem zu betrachtenden Auge gehalten, beispielsweise mittels einer indirekten Ophthalmoskopierlupe im "non-contact"-Betrieb.

Abhängig von den optischen Eigenschaften der Ophthalmoskopierlupe beziehungsweise des Kontaktglases lassen sich auf diese Weise große, weitwinklige Bereiche des Augenhintergrundes, etwa durch Lupen/Gläser mit hoher Brechkraft, oder kleinere aber dafür hoch aufgelöste Bereiche des Augenhintergrundes, etwa durch Lupen/Gläser mit geringer Brechkraft, betrachten. Für eine optimale beziehungsweise formatfüllende Abbildung ist jede Ophthalmoskopierlupe beziehungsweise jedes Kontaktglas in Abhängigkeit von dessen Brechkraft in einem bestimmten Abstand zum Auge zu positionieren, da ansonsten Vignettierung durch Iris oder ähnliches auftreten kann.

Verschiedene Randbedingungen während einer Operation, wie beispielsweise das Beschlagen der Ophthalmoskopierlupe, die Zugänglichkeit und der Arbeitsraum in der Nähe des Patientenauges, und dergleichen machen unter Umständen, insbesondere bei sehr kurzbrennweitigen Ophthalmoskopierlupen, die in der Regel sehr dicht zum Auge hin positioniert werden müssen, ein Verschieben der Ophthalmoskopierlupe vom Auge weg erforderlich. Dadurch verschiebt sich die Lage des (Zwischen-)Bildes und es kommt zusätzlich zu Vignettierung.

In dem deutschen Gebrauchsmuster G 94 15 219 U1 ist beispielsweise eine solche Vorsatzeinrichtung für ein Mikroskop beschrieben. Bei dem Mikroskop handelt es sich um ein Operationsmikroskop für die Ophthalmologie zur Beobachtung eines Auges. Das Mikroskop weist unter anderem ein Hauptobjektiv auf, in dessen Umgebung die Vorsatzeinrichtung befestigt ist. Die bekannte Vorsatzeinrichtung weist eine Halteeinrichtung in Form eines Haltearms auf, an der/dem ein Linsenelement angeordnet ist, welches wahlweise in den durch das Objektiv hindurch verlaufenden Strahlengang eingeschwenkt werden kann. Dazu ist der Haltearm an einer Positioniereinrichtung befestigt, bei der es sich um ein Gestänge handelt. Zunächst kann der Haltearm, und mit ihm des Linsenelement, um das Gestänge herum geschwenkt werden. Weiterhin ist noch ein Linearantrieb vorgesehen. Über diesen Linearantrieb kann das Gestänge parallel zur optischen Achse in Längsrichtung verfahren werden. Über das Gestänge kann der Haltearm und mit ihm das Linsenelement somit auch noch parallel der optischen Achse in Längsrichtung verfahren werden, so dass die Position des Linsenelements auf der optischen Achse zwischen Objektiv und zu beobachtendem Auge verschoben werden kann. Die Positioniereinrichtung ist über eine Befestigungseinrichtung, die bei der bekannten Lösung als Adapter ausgebildet ist, an dem Mikroskop befestigt. Die Befestigungseinrichtung ist dabei in einer Weise ausgebildet, dass die gesamte Vorsatzeinrichtung aus dem Strahlengang herausgeschwenkt werden kann.

Während in der G 94 15 219 U1 eine Vorsatzeinrichtung beschrieben ist, über die es möglich ist, ein einzelnes Linsenelement wahlweise in den Strahlengang einzubringen, ist in der US 6,943,942 B eine Vorsatzeinrichtung mit einem ähnlichen Aufbau beschrieben, mit der es auch möglich ist, wahlweise zwei Linsenelemente in den Strahlengang einzubringen. Diese bekannte Lösung sieht vor, dass zwei Linsenelemente vorhanden sind, die jeweils über eine eigene Halteeinrichtung in Form eines Hebelarms in der oben geschilderten Weise an einer Positioniereinrichtung befestigt sind. Diese bekannte Lösung zielt darauf ab, dass temporär eine zweite Linse zusätzlich in den Strahlengang des Mikroskops geschwenkt werden kann, um die Brechkraft der Linsenelemente derart zu manipulieren, dass sich der vordere Augenabschnitt betrachten lässt.

Die bisher bekannten Lösungen weisen allerdings eine Reihe von Nachteilen auf. So wird für eine bekannte Vorsatzeinrichtung, wie sie beispielsweise in der US 5,793,524 B beschrieben ist, ein relativ großer Bauraum benötigt, da eine Linerarverschiebung der Linsenelemente in Längsrichtung, und damit parallel zur optischen Achse durchgeführt werden muss. Ein großer Nachteil ist die Fokussierung über eine Linearverschiebung des Linsenelements relativ zur Beobachtungseinrichtung und somit zum Patientenauge. Beim Fokussieren wird gleichzeitig auch die Pupillenlage verändert. Wenn beispielsweise die Pupillenlage der Beobachtungseinrichtung zu weit von der Eintrittspupille des Patientenauges entfernt liegt, kommt es zu unerwünschter Vignettierung, da der sichtbare Bereich des Augenhintergrundes kleiner wird. Das Einrichten und Fokussieren ist somit ein iterativer Prozess. Selbst wenn das Linsenelement aus dem Strahlengang herausgeschwenkt ist, oder sich in einer Position nahe des Objektivs befindet, kann der Bauraum nicht verringert werden, da das Positioniergestänge und der Linearantrieb in ihrer Längsausdehnung und Positionierung unverändert bleiben. Die Vorsatzeinrichtung an sich ist wegen des Linearantriebs ebenfalls relativ großvolumig ausgebildet. Auch dies hat verschiedene Nachteile. Wenn beispielsweise die Vorsatzeinrichtung komplett verschwenkt wird, wie dies bei der G 94 15 219 U1 als möglich beschrieben wird, ist ein beträchtlicher Leerraum erforderlich, in den die Vorsatzeinrichtung eingeschwenkt werden kann. Weiterhin ist es auch schwierig, die Vorsatzeinrichtung zu reinigen und gegebenenfalls zu sterilisieren. Werden Mikroskope als Operationsmikroskope eingesetzt, so müssen die Vorsatzeinrichtungen nach dem Gebrauch sterilisiert werden, was in der Regel in einem Autoklaven geschieht. Große, komplexe und sperrige Bauteile sind dabei nur sehr schwer in einem Autoklaven sterilisierbar.

Weiterhin sind die bekannten Lösungen konstruktiv aufwändig und damit teuer, da insbesondere ein separater Antrieb vorgehalten werden muss. Derartige Antriebe, die zumeist elektrisch funktionieren, können zusätzlich anfällig für Störungen sein.

Ausgehend vom genannten Stand der Technik liegt der vorliegenden Erfindung die Aufgabe zugrunde, eine Vorsatzeinrichtung der eingangs genannten Art derart weiterzubilden, dass diese auf konstruktiv einfache und kostengünstige Weise realisierbar ist. Darüber hinaus soll mittels der erfindungsgemäßen Vorsatzeinrichtung auch eine verbesserte Handhabung und Bedienbarkeit erreicht werden. Weiterhin soll eine entsprechend verbesserte Beobachtungseinrichtung bereitgestellt werden.

Diese Aufgabe wird erfindungsgemäß gelöst durch die Vorsatzeinrichtung mit den Merkmalen gemäß dem unabhängigen Patentanspruch 1. Weitere Merkmale und Details der Erfindung ergeben sich aus den Unteransprüchen, der Beschreibung sowie den Zeichnungen. Dabei gelten Merkmale und Details, die im Zusammenhang mit der erfindungsgemäßen Vorsatzeinrichtung beschrieben sind, selbstverständlich auch im Zusammenhang mit der erfindungsgemäßen optischen Beobachtungseinrichtung, und jeweils umgekehrt, so dass wechselweise jeweils auf die entsprechenden Ausführungen verwiesen und vollinhaltlich Bezug genommen wird. Gleiches gilt in entsprechender Weise bezüglich der erfindungsgemäßen Revolvereinrichtung.

Gemäß der vorliegenden Erfindung wird zunächst eine verbesserte Vorsatzeinrichtung für ein Operationsmikroskop bereitgestellt.

Die erfindungsgemäße Vorsatzeinrichtung ist konstruktiv einfach herstellbar und platzsparend ausgebildet, insbesondere dann, wenn das Linsenelement nicht benötigt wird. Diese Eigenschaft ist vor allem auch dann von Vorteil, wenn die Vorsatzeinrichtung gereinigt werden muss, beispielsweise in einem Autoklaven. Darüber hinaus ist die Vorsatzeinrichtung leicht zu handhaben und zu bedienen.

Durch die vorliegende Erfindung wird eine Vorsatzeinrichtung bereitgestellt, die für ein Operationsmikroskop, insbesondere ein Operationsmikroskop für die Ophthalmologie, verwendet werden kann.

Unter einer Vorsatzeinrichtung soll dabei eine Einrichtung verstanden werden, die vor wenigstens einem bestimmten Bauteil der optischen Beobachtungseinrichtung angeordnet wird. Wenn es sich bei der optischen Beobachtungseinrichtung beispielsweise um ein Mikroskop handelt, verfügt dieses in der Regel über ein Objektivelement. Vorteilhaft kann eine Vorsatzeinrichtung in einem solchen Fall dazu gedacht sein, vor dem Objektiv oder im Umgebungsbereich des Objektivs angebracht zu werden, um insbesondere einen durch das Objektiv hindurch verlaufenden Strahlengang zu beeinflussen. Beispielsweise kann es sich bei der Vorsatzeinrichtung um ein separates Gerät handeln, dass unabhängig von der optischen Beobachtungseinrichtung hergestellt wird, und um das die optische Beobachtungseinrichtung je nach Bedarf ergänzt werden kann. Natürlich kann die Vorsatzeinrichtung auch als fester Bestandteil der optischen Beobachtungseinrichtung ausgebildet sein. Wenn die Vorsatzeinrichtung zwischenzeitlich gereinigt werden muss, ist vorteilhaft, wenn diese lösbar an der optischen Beobachtungseinrichtung angeordnet ist.

Die erfindungsgemäße Vorsatzeinrichtung weist zunächst eine Halteeinrichtung auf. Die Halteeinrichtung weist ein Halteelement auf, an welchem wenigstens ein Linsenelement angeordnet ist. Dabei ist die Erfindung generell nicht auf bestimmte Ausgestaltungsformen für die Halteeinrichtung und das Halteelement beschränkt. Ebenso ist die Erfindung generell nicht auf eine bestimmte Anzahl von Linsenelementen, auf bestimmte Linsenelementtypen oder bestimmte Anordnungspositionen und Anordnungsarten der Linsenelemente an dem Halteelement beschränkt. Verschiedene vorteilhafte, allerdings nicht ausschließliche Beispiele hierzu werden im weiteren Verlauf der Beschreibung näher erläutert.

Das Halteelement kann vorteilhaft als eine Art Revolvereinrichtung ausgebildet oder Bestandteil einer solchen Revolvereinrichtung sein. Eine derartige Revolvereinrichtung wird im weiteren Verlauf der Beschreibung noch näher erläutert. Die Revolvereinrichtung dient generell dazu, dass die Linsenelemente in einen Strahlengang eingeschwenkt werden können. Besonders vorteilhaft ist gemäß der vorliegenden Erfindung vorgesehen, dass immer nur ein Linsenelement aus einer Auswahl von Linsenelementen in den Strahlengang eingeschwenkt wird, und zwar durch entsprechende Drehung der Revolvereinrichtung.

Ein weiteres grundsätzliches Merkmal der erfindungsgemäßen Vorsatzeinrichtung wird durch eine Positioniereinrichtung gebildet. Zunächst zeichnet sich die Positioniereinrichtung dadurch aus, dass an dieser das Halteelement für das wenigstens eine Linsenelement angeordnet ist. Dabei ist die Erfindung nicht auf bestimmte Ausführungsformen beschränkt, wo und wie das Halteelement an der Positioniereinrichtung zu befestigen ist. Einige vorteilhafte, jedoch nicht ausschließliche Beispiele hierzu werden im weiteren Verlauf der Beschreibung näher erläutert.

Die Positioniereinrichtung dient zum Positionieren des Halteelements und des daran angeordneten wenigstens einen Linsenelements in Bezug auf eine optische Beobachtungseinrichtung und/oder eine Fokussiereinrichtung, die weiter unten noch näher beschrieben wird. Das bedeutet, dass das Halteelement und das daran angeordnete wenigstens eine Linsenelement über die Positioniereinrichtung in eine gewünschte Position verbracht werden können, in der insbesondere das Linsenelement seine vorgesehene Funktion erfüllen kann. Je nach Einsatzzweck und Einsatzgebiet der Vorsatzeinrichtung können unterschiedliche Anforderungen an die Positioniereinrichtung und deren Ausgestaltungen gestellt werden.

Vorteilhaft ist vorgesehen, dass die Positioniereinrichtung in einer Weise ausgebildet ist, dass mit dieser diskrete Positionierzustände eingestellt werden können. Vorzugsweise ist vorgesehen, dass mittels der Positioniereinrichtung lediglich zwei Positionierzustände eingestellt werden können, nämlich eine Parkposition (beispielsweise in einem zusammengeklappten Zustand) und eine Arbeitsposition (in einem auseinander geklappten Zustand).

Wie eine Positionierung in Bezug auf die optische Beobachtungseinrichtung und/oder die Fokussiereinrichtung erfolgen kann, soll anhand eines Beispiels exemplarisch verdeutlicht werden, ohne dass die Erfindung auf dieses konkrete Beispiel beschränkt wäre. Beispielsweise kann vorgesehen sein, dass es sich bei der optischen Beobachtungseinrichtung um ein wie bereits erwähntes Mikroskop mit Objektiv handelt. Wenn nun die Vorsatzeinrichtung dazu dienen soll, dass ein zusätzliches Linsenelement wahlweise in den Strahlengang, der durch das Objektiv hindurch verläuft, eingebracht werden soll, ist es die Funktion der Positioniereinrichtung, das Halteelement und das daran angeordnete wenigstens eine Linsenelement so vor dem Objektiv und damit in Bezug zu diesem zu platzieren, dass das Linsenelement in den Strahlengang eingebracht werden kann.

Das Einsatzgebiet der Vorsatzeinrichtung liegt bevorzugt in der indirekten kontaktlosen Ophthalmologie, bei Bedarf auch in der Unterstützung der indirekten kontaktbehafteten Ophthalmologie, insbesondere unter Verwendung einer Fokussiereinrichtung. Sie dient vorteilhaft zur Betrachtung eines Zwischenbildes mittels einer optischen Beobachtungseinrichtung, bevorzugt mittels eines stereoskopischen Operationsmikroskops der Ophthalmologie, während eines chirurgischen Eingriffes am Auge, insbesondere auf dem Gebiet der Hinterabschnittschirurgie.

Gemäß dem ersten Aspekt der Erfindung wird eine Vorsatzeinrichtung für ein Operationsmikroskop bereitgestellt, mit einer Fokussiereinrichtung und mit einer Halteeinrichtung, aufweisend ein Halteelement, an welchem wenigstens ein Linsenelement angeordnet ist, sowie weiter aufweisend eine Positioniereinrichtung zum Positionieren des Halteelements und des daran angeordneten wenigstens einen Linsenelements in Bezug auf die Fokussiereinrichtung, wobei das Halteelement an der Positioniereinrichtung angeordnet ist. Die Vorsatzeinrichtung ist erfindungsgemäß **dadurch gekennzeichnet, dass** wenigstens eine Abdeckkappe für die Fokussiereinrichtung vorgesehen ist, an der die Halteeinrichtung über eine Befestigungseinrichtung befestigt ist und dass die wenigstens eine Abdeckkappe mit der Fokussiereinrichtung verbunden ist.

Neben den zuvor beschriebenen Grundmerkmalen der Vorsatzeinrichtung weist die Vorsatzeinrichtung gemäß dem ersten Aspekt weiterhin eine Fokussiereinrichtung auf. Bei der Fokussiereinrichtung handelt es sich generell um eine Fokussieroptik, die beispielsweise als Reduzieroptik ausgebildet ist.

Vorteilhaft ist vorgesehen, dass die Fokussiereinrichtung als ein Bestandteil der Vorsatzeinrichtung ausgebildet ist. Bevorzugt handelt es sich dabei um ein separates Bauteil, welches einen Bestandteil der Vorsatzeinrichtung darstellt. Natürlich lässt sich die Fokussiereinrichtung beziehungsweise lassen sich deren Funktionen auch in die optische Beobachtungseinrichtung, etwa in ein Mikroskop, integrieren. Ein solches Beispiel ist im Zusammenhang mit der optischen Beobachtungseinrichtung weiter unten näher erläutert.

Vorteilhaft kann die Vorsatzeinrichtung über die Fokussiereinrichtung an der optischen Beobachtungseinrichtung befestigt werden oder sein. Hier ist insbesondere eine lösbare Verbindung bevorzugt. Wie dies im Einzelnen realisiert werden kann, wird anhand einiger vorteilhafter, jedoch nicht ausschließlicher Beispiele weiter unten in größerem Detail beschrieben.

Üblicherweise ist die Fokussiereinrichtung nicht steril beziehungsweise nicht sterilisierbar, da eine Sterilisierbarkeit oftmals nicht erforderlich/gewünscht ist. Bei einem Operationsmikroskop ist indes eine Sterilisierung nach dem Gebrauch erforderlich. Zu diesem Zweck weist die erfindungsgemäße Vorsatzeinrichtung wenigstens eine Abdeckkappe für die Fokussiereinrichtung auf. Die Abdeckkappe ist mit der Fokussiereinrichtung verbunden, so dass insbesondere Bedienelemente und berührungsgefährdete Bereiche der Fokussiereinrichtung durch - insbesondere sterilisierbare - Abdeckkappen geschützt sind. Das hat den Vorteil, dass komplexe, aufwendige, empfindliche Bauelemente nicht der hohen Belastung von Reinigung, Desinfektion, Sterilisation ausgesetzt sind. In der Fokussiereinrichtung können unproblematisch entsprechend komplexe beziehungsweise auch empfindliche mechanische und optische Bauteile / Baugruppen verbaut sowie Elektronik integriert und motorische Antriebe verwendet werden, zum Beispiel motorische Funktionen wie Fokussierung; Steuerungs- oder Überwachungsfunktionen, und dergleichen.

Da die Fokussiereinrichtung von der Abdeckkappe abgedeckt wird, braucht die Fokussiereinrichtung nicht sterilisiert zu werden. Vielmehr reicht es dann aus, wenn die Abdeckkappe sterilisiert wird. Die Abdeckkappe hat insbesondere den Vorteil, dass diese Einheit sterilisierbar ist. Eine Sterilisierbarkeit ist bei Einsatz zu Operationszwecken zwingend erforderlich. Die sterile Kappe bietet Schutz beziehungsweise Abdeckung der berührungsgefährdeten Bereiche an der Fokussiereinrichtung, die einen unsterilen Bereich darstellt, und ermöglicht somit die sterile Bedienung der Vorsatzeinrichtung während der Operation. Um den sterilen Betrieb der Vorsatzvorrichtung zu gewährleisten, wird eine relativ einfache und kostengünstige Baugruppe verwendet, wobei die Reinigung, Desinfektion, Sterilisation dieser Baugruppe wesentlich einfacher ist als die Reinigung, Desinfektion, Sterilisation der gesamten Vorsatzeinrichtung. Bei der Durchführung von mehreren chirurgischen Eingriffen nacheinander werden mehrere sterile Systeme benötigt. Hierfür müssen mehrere Sätze steriler Abdeckkappen und Linsenelemente, beispielsweise Ophthalmoskopierlupen, bereit stehen beziehungsweise verfügbar sein, und nicht komplette Systeme mit sterilisierter Fokussier-/Reduzieroptik und Antriebseinheit. Die sterile Abdeckkappe lässt sich temporär auf den, vorteilhaft verschiebbaren, Bereich der Fokussiereinrichtung unter sterilen Bedingungen während der Operation, bei Bedarf auch mehrmals, aufstecken. Beim Aufstecken der Abdeckkappe auf der Fokussiereinrichtung kann die Abdeckkappe beispielsweise so verrasten, dass die Abdeckkappe zur Fokussiereinrichtung ausgerichtet und gegen ungewolltes Lösen von der Fokussiereinrichtung während der Operation gesichert ist. Ein Lösen, beispielsweise Entrasten der Abdeckkappe, beispielsweise über zwei Bedienelemente /Druckbereiche an der Kappe, und somit ein Entfernen der Abdeckkappe von der Fokussiereinrichtung unter sterilen Bedingungen während der Operation, ist möglich. Das Aufschieben der Abdeckkappe, insbesondere der sterilen Kappe, auf den, beispielsweise verschiebbaren, Bereich der Fokussiereinrichtung erfolgt vorteilhaft über Formpaarung. Eine Fixierung, beispielsweise eine Verrastung der Abdeckkappe zur Fokussiereinrichtung kann beispielsweise über zwei angefederte Rastelemente an der Fokussiereinrichtung erfolgen, die jeweils in einen Hinterschnitt an der sterilen Kappe der Abdeckkappe eingreifen. Zum Entrasten und Abnehmen der Abdeckkappe von der Fokussiereinrichtung werden dann vorteilhaft zwei verformbare, elastische Bereiche an der sterilen Abdeckkappe derart verformt, dass damit die Rastelemente der Fokussiereinrichtung bewegt und somit die Kappe entrastet wird und von der Fokussiereinrichtung abgezogen werden kann.

Neben der Abdeckung der Fokussiereinrichtung dient die Abdeckkappe auch dazu, dass an dieser die Halteeinrichtung über eine Befestigungsvorrichtung befestigt ist. Des Weiteren verfügt die Vorsatzeinrichtung folglich über eine Befestigungseinrichtung, mittels derer die Halteeinrichtung an der Abdeckkappe befestigt werden kann. Wie dies im Einzelnen realisiert werden kann wird im weiteren Verlauf der Beschreibung, insbesondere auch im Zusammenhang mit der weiter untern beschriebenen erfindungsgemäßen optischen Beobachtungseinrichtung, näher erläutert. Beispielsweise kann die Befestigungseinrichtung derart ausgebildet sein, dass eine lösbare und/oder schwenkbare und/oder drehbare und/oder linear verschiebbare Anbindung realisiert werden kann.

Eine Vorsatzeinrichtung gemäß der vorliegenden Erfindung kann beispielsweise aus drei Grundkomponenten bestehen, einer Fokussiereinrichtung, einer sterilen Abdeckkappe und Halteeinrichtung für optische Elemente zur Abbildung des Augenhintergrundes, etwa zur Erzeugung eines Zwischenbildes, und mindestens einem Linsenelement, etwa einer Ophthalmoskopierlupe. Bei Verwendung eines Linsenwechslers, hierbei handelt es sich um das Halteelement der Halteeinrichtung, an der Abdeckkappe können auch mehrere Linsenelemente, beispielsweise Ophthalmoskopierlupen, verwendet werden.

Ein bevorzugtes Merkmal der vorliegenden Erfindung ist, dass die Abdeckkappe lösbar mit der Fokussiereinrichtung verbunden ist. Über eine solche Verbindung, etwa eine geeignete Koppelstelle an der Fokussiereinrichtung, lässt sich die Abdeckkappe temporär, das heißt während eines chirurgischen Eingriffes, bei Bedarf auch mehrmals, auf-/abstecken, und zwar unter sterilen Bedingungen. Ein Fixiermechanismus, beispielsweise ein Verrastungsmechanismus, für die Abdeckkappe ist vorteilhaft ebenfalls in die Fokussiereinrichtung integriert.

Vorteilhaft kann die Fokussiereinrichtung wenigstens ein optisches Bauelement zur Fokussierung einer Beobachtungseinrichtung auf das Zwischenbild des wenigstens einen Linsenelements aufweisen.

Die Fokussiereinrichtung enthält zusätzliche optische Bauelemente zur Fokussierung der Beobachtungsvorrichtung, im Folgenden auch Fokussieroptik genannt, auf das Zwischenbild des Linsenelements, beispielsweise einer indirekten Ophthalmoskopierlupe, die im "non-contact" oder "contact"-Modus betrieben wird. Die Fokussieroptik besteht vorteilhaft aus wenigstens einem, insbesondere festen, Negativglied und wenigstens einem, insbesondere verschiebbaren, Positivglied. Die Fokussierung kann manuell und/oder motorisch erfolgen. Die Fokussieroptik ist vorteilhaft so ausgelegt, dass sich die verwendete Beobachtungsvorrichtung, beispielsweise das Mikroskop, mit der Brennweite f auf einen Bereich f1...f2 fokussieren lässt. Typisch ist f1≈f-7mm & f2≈f-50mm; ideal f1=0 & f2=f-60mm; der Bereich f1...f2 deckt dabei die Zwischenbildebenen für typische Ophthalmoskopierlupen und Kontaktgläser, etwa 40D...130D unter den üblichen Anwendungsbedingungen ab, wie typische Abstände der Ophthalmoskopierlupe zum Patientenauge; Phakie/Pseudophakie/ Aphakie; Augapfel mit natürlichem Glaskörper/ölgefüllt/gasgefüllt; kurz-/weitsichtiges Patientenauge; Betrachtung der Retina bzw. Bereiche im Glaskörper im Abstand x über der Retina; und dergleichen.

Damit ist die Fokussierbewegung von der Bewegung des Linsenelements, beispielsweise der Ophthalmoskopierlupe, zum Patientenauge entkoppelt. Der Abstand der Ophthalmoskopierlupe zum Patientenauge beeinflusst in der Regel den sichtbaren Bereich des Augenhintergrundes vom Zwischenbild. Es wird nunmehr eine optimale sterile Abdeckkappe und Haltevorrichtung bereitgestellt. Das System lässt sich also wesentlich einfacher einrichten, etwa hinsichtlich einer Positionierung der Ophthalmoskopierlupe bezüglich des Patientenauges, und fokussieren. Somit ist bei entsprechender Positionierung des Linsenelements, beispielsweise einer Ophthalmoskopierlupe beziehungsweise eines Kontaktglases, eine Betrachtung des Augenhintergrundes unter verschiedensten Bedingungen und für unterschiedliche Linsenelemente ohne Positionsänderungen an der Beobachtungsvorrichtung möglich, wobei der Abstand Patientenauge zu Mikroskop unverändert bleibt. Daraus resultiert ein schneller und unkomplizierter Wechsel zwischen Betrachtung des Augenvordergrundes und Augenhintergrundes und eine Reduzierung der Gefahr einer Kollision der Vorrichtung mit dem Patienten(-auge).

Ein Operateur hat aber weiterhin über die Verschiebung des Gesamtsystems die Möglichkeit, bei Bedarf den Abstand der Ophthalmoskopierlupe zum Patientenauge bewusst zu manipulieren. Dies ist beispielsweise dann von Interesse, wenn das Linsenelement zu dicht am Patientenauge ist und somit bei der Arbeit mit den chirurgischen Instrumenten im Auge stört oder beschlägt; dies ist vor allem bei kurzbrennweitigen, also hochbrechenden, Ophthalmoskopierlupen der Fall.

Nachfolgend wird nun die Halteeinrichtung in größerem Detail erläutert.

Vorteilhaft kann vorgesehen sein, dass die Halteeinrichtung lösbar an der Abdeckkappe angeordnet ist. In weiterer Ausgestaltung kann alternativ oder zusätzlich vorgesehen sein, dass die Halteeinrichtung drehbar und/oder schwenkbar und/oder linear verschiebbar an der Abdeckkappe angeordnet ist.

Vorzugsweise ist vorgesehen, dass die Positioniereinrichtung der Vorsatzeinrichtung in besonderer Weise ausgestaltet ist. Dazu ist vorteilhaft vorgesehen, dass die Positioniereinrichtung wenigstens zwei Positionierglieder aufweist, die über ein Gelenk miteinander verbunden sind. Im einfachsten Fall ist es ausreichend, wenn zwei Positionierglieder vorgesehen sind, die über ein Gelenk miteinander verbunden sind. Natürlich sind auch Anwendungsfälle denkbar, in denen die Positioniereinrichtung mehr als zwei Positionierglieder aufweist, wobei zwischen jeweils zwei benachbarten Positioniergliedern jeweils ein Gelenkelement vorgesehen ist.

Generell ist die Erfindung nicht auf bestimmte Ausgestaltungsformen für die Positionierglieder beschränkt. Einige vorteilhafte, jedoch nicht ausschließliche Beispiele hierzu werden im weiteren Verlauf der Beschreibung näher erläutert. Ebenso ist die Erfindung nicht auf den Einsatz bestimmter Gelenktypen beschränkt. Unter einem Gelenk ist im vorliegenden Fall ganz allgemein eine bewegliche Verbindung zwischen zwei Körpern zu verstehen, wobei es sich bei den Körpern um die Positionierglieder handelt. Einige vorteilhafte, jedoch nicht ausschließliche Beispiele geeigneter Gelenktypen werden im weiteren Verlauf der Beschreibung näher erläutert.

Die erfindungsgemäße Vorsatzeinrichtung weist im Vergleich zu den aus dem Stand der Technik bekannten Lösungen eine Reihe von Vorteilen auf. So ist die Vorsatzeinrichtung zunächst konstruktiv einfach herstellbar, da insbesondere auf aufwändige Konstruktionen wie Linearantriebe oder dergleichen verzichtet werden kann. Ebenso kann der für die Vorsatzeinrichtung erforderliche Bauraum reduziert werden, so dass die Vorsatzeinrichtung zum einen in platzsparender Weise an einer optischen Beobachtungseinrichtung angebracht werden kann. Dies gilt insbesondere dann, wenn das an der Vorsatzeinrichtung angeordnete wenigstens eine Linsenelement einmal nicht benötigt wird. Des Weiteren hat die platzsparende Ausgestaltung auch den Vorteil, dass die Vorsatzeinrichtung, sofern erforderlich, auf einfache Weise gereinigt werden kann. Eine Reinigung kann dabei in einer Weise erfolgen, dass zunächst eine mechanische Reinigung erfolgt, beispielsweise in wenigstens einer Spüleinrichtung, gefolgt von einer Desinfektion in einem Lösungsbad sowie einer Sterilisation in einem Autoklaven.

Vorteilhaft kann bei einer solchen Ausführungsform die Halteeinrichtung lösbar an der Vorsatzeinrichtung angeordnet sein. In weiterer Ausgestaltung kann die Halteeinrichtung alternativ oder zusätzlich drehbar und/oder schwenkbar und/oder linear verschiebbar an der Vorsatzeinrichtung angeordnet sein.

Wie weiter oben schon ausgeführt wurde, ist die Erfindung nicht auf bestimmte Gelenktypen für die Verbindung der Positionierglieder beschränkt. Nachfolgend werden hierzu einige vorteilhafte Ausgestaltungen genannt. Gemäß einer bevorzugten Ausgestaltung können die Positionierglieder über ein Drehgelenk miteinander verbunden sein. Ein Drehgelenk zeichnet sich allgemein dadurch aus, dass die zwei Positionierglieder über dieses Drehgelenk gegeneinander verdreht werden können. Vorteilhaft ist dabei vorgesehen, dass das Drehgelenk aus zwei Gelenkelementen besteht, die jeweils an einem Ende des Positionierglieds vorgesehen sind. Beispielsweise könnte ein solches Drehgelenk realisiert werden, in dem in den zu verbindenden Enden der Positionierglieder Bohrungen oder allgemein Öffnungen vorgesehen sind, durch die ein Bolzenelement hindurchgeführt ist. Die Positionierglieder drehen bei dieser Ausgestaltung um eine gemeinsame Drehachse. In ähnlicher Weise könnte ein Gelenk beispielsweise auch als Schraubgelenk, als Scharniergelenk oder dergleichen ausgestaltet sein. Ebenso ist natürlich auch denkbar, dass das Gelenk als Kugelgelenk ausgebildet ist. Verfügt die Positioniereinrichtung über mehrere Positionierglieder und somit auch über mehrere Gelenke, können jeweils Gelenke desselben Typs, aber auch Gelenke unterschiedlichen Typs zum Einsatz kommen.

Vorteilhaft ist vorgesehen, dass die Gelenke in einer Weise ausgebildet sind, dass sie die Drehung des einen Positionierglieds in Bezug auf das andere Positionierglied um einen bestimmten Drehwinkel zulassen. Bei dem Drehwinkel handelt es sich dann um den Öffnungswinkel der beiden Positionierglieder. Vorteilhaft kann dabei vorgesehen sein, dass das Gelenkelement derart ausgestaltet ist, dass damit ein Drehwinkel beziehungsweise ein Öffnungswinkel der Positionierglieder von kleiner 90 Grad, vorzugsweise in einem Bereich zwischen 40 und 80 Grad, ermöglicht werden kann. Der Grund hierfür ist beispielsweise eine Sicherheitsfunktion, um einen unerwünschten Kontakt mit dem Patienten(-auge) zu vermeiden. Der Drehwinkel beeinflusst grundsätzlich die Lage der Linsenelemente. Deshalb ist es bevorzugt, dass über das Gelenk solch eine Drehung zugelassen wird, dass eine definierte reproduzierbare Endlage und Ruhelage (beispielsweise in einem zusammengeklappten Zustand) realisierbar ist.

In bevorzugter Ausgestaltung kann vorgesehen sein, dass die Vorsatzeinrichtung eine Positioniereinrichtung mit zwei Positioniergliedern aufweist, dass ein erstes Positionierglied über eine Befestigungseinrichtung in wenigstens einer ersten Schwenkrichtung schwenkbar an der optischen Beobachtungseinrichtung oder an der Vorsatzeinrichtung oder an der Abdeckkappe angeordnet ist, und dass ein zweites Positionierglied über ein Gelenk in wenigstens einer, von der wenigstens einen ersten Schwenkrichtung abweichenden, zweiten Schwenkrichtung schwenkbar am ersten Positionierglied angeordnet ist.

Über ein Drehgelenk lässt sich die Positioniereinrichtung um die optische Achse drehen. Ein eingeschwenktes Linsenelement bleibt dabei weiterhin koaxial zur optischen Achse. Die Lage der Positionierglieder, die eine Art Scherenmimik darstellen, sowie die Lage des Halteelements lassen sich somit während des chirurgischen Eingriffs, insbesondere bei Arbeiten am Hinterabschnitt, den Arbeitsraumbedingungen und Arbeitsraumwünschen des Operateurs anpassen.

Über die Positionierglieder, die in einer solchen Anordnung eine Art Scherenmechanismus bilden, die dann vorzugsweise zwei definierte Endpositionen, nämlich "Rastposition zusammengefaltet" und "Endanschlag Gravitation auseinandergefaltet", aufweisen, kann das Halteelement und mit ihm jedes daran befindliche Linsenelement definiert positioniert werden. Bei auseinander gefalteten Positioniergliedern, die vorteilhaft durch Gravitation in dieser Position auf Endanschläge gehalten werden, und eingeschwenktem Halteelement wird das Linsenelement, vorteilhaft über eine typspezifische Fassung, in einem definierten, festen Abstand zur Beobachtungseinrichtung und koaxial zur optischen Achse positioniert. Dabei sind Linsenelementfassung und Linsenelementhalter vorteilhaft so auf die verwendete Beobachtungsvorrichtung abgestimmt, dass das jeweilige Linsenelement im ausgeschwenkten Zustand einen optimalen Abstand, beispielsweise für eine formatfüllende Abbildung des Augenhintergrundes - ohne Vignettierung, zum Patientenauge einnimmt, wenn die Beobachtungseinrichtung vor Einschwenken des Linsenelements und der Fokussiereinrichtung auf die Ebene "Iris Patientenauge" fokussiert war. Damit ist sichergestellt, dass die Lage der Eintrittspupille der Beobachtungseinrichtung und die Pupille des Patientenauges nahe beieinander liegen.

Das Falten des Linsenelementhalters vergrößert den Sicherheitsbereich, etwa den Freilauf, im Falle einer Kollision der Ophthalmoskopierlupe mit dem Patienten(-auge) aufgrund einer ungewollten beziehungsweise fehlerhaften Bewegung des Gesamtsystems.

Die Linsenelementhaltermimik lässt sich kompakt, insbesondere bauraumminimiert, in einander falten, wodurch weniger Störkonturen entstehen. Außerdem sind Fokussieroptik und Halteelement und Linsenelementfassung aufeinander abgestimmt und führen zu den oben genannten applikativen Vorteilen, wie beispielsweise Entkopplung der Bewegungen für Pupillenlage - sichtbarer Bereich - und Fokus. Darüber hinaus wird die Kollisionsgefahr mit Patienten herabgesetzt, da kein Verfahren des Gesamtsystems mehr erforderlich ist. Auch ist ein schneller Wechsel zwischen Vorder- und Hinterabschnitt möglich.

Über ein zusätzliches Drehgelenk und eine spezielle Linsenelementaufnahme, beispielsweise eine Aufnahme für mehrere verschiedene Ophthalmoskopierlupen, lassen sich kurzfristig wechselseitig verschiedene Ophthalmoskopierlupen in den Strahlengang der Optik, auch während einer Operation, auch mehrmals, auch unter sterilen Bedingungen, einschwenken oder über eine entsprechende Zwischenstellung sämtliche Ophthalmoskopierlupen aus dem Strahlengang ausschwenken. Der Linsenelementwechsler, das Halteelement, ist vorteilhaft so gestaltet, dass die jeweilige(n) Ophthalmoskopierlupe(n), welche nicht benötigt wird/werden, den Bauraum unterhalb der Beobachtungsvorrichtung möglichst so gering wie möglich stören. Dies wird vorteilhaft erreicht durch eine entsprechende winklige Anordnung der Drehgelenke und Kröpfung der Linsenelementfassungen.

Die Verwendung eines Ophthalmoskopierlupen-Wechslers, eines Halteelements, ermöglicht das wechselseitige, nicht gleichzeitige, Einschwenken verschiedener Ophthalmoskopierlupen sowie das Ausschwenken der (aller) Ophthalmoskopierlupen aus dem Strahlengang der Beobachtungsvorrichtung bei eingeschwenkter Fokussieroptik, vorzugsweise bei Verwendung von Kontaktgläsern.

Das Halteelement und die Linsenelemente, insbesondere deren Fassung, sind dabei vorteilhaft so gestaltet, dass die Linsenelemente abhängig von ihrer Brechkraft in unterschiedlichen, definierten Abständen zur Beobachtungsvorrichtung beziehungsweise zu dem Patientenauge positioniert werden können.

Wie weiter oben schon erwähnt wurde, kann/können die Positioniereinrichtung, und insbesondere deren Positionierglieder, auf unterschiedliche Weise ausgebildet sein. Nachfolgend werden hierzu einige vorteilhafte, jedoch nicht ausschließliche Ausführungsformen beschrieben. Vorteilhaft kann vorgesehen sein, dass die Positioniereinrichtung ein erstes Positionierglied aufweist, das an seinem einen Ende ein Gelenkelement zur gelenkigen Verbindung mit einem zweiten Positionierglied aufweist, und dass am anderen Ende des ersten Positionierglieds die Befestigungseinrichtung vorgesehen ist. Vorteilhaft kann in weiterer Ausgestaltung alternativ oder zusätzlich vorgesehen sein, dass die Positioniereinrichtung ein zweites Positionierglied aufweist, das an seinem einen Ende ein Gelenkelement zur gelenkigen Verbindung mit einem ersten Positionierelement aufweist, und dass am anderen Ende des zweiten Positionierglieds die Halteeinrichtung angeordnet ist. Beispielsweise können die Positionierglieder in einem solchen Fall in Form eines lang gestreckten Körpers ausgebildet sein und insbesondere eine stabförmige Kontur aufweisen.

Vorzugsweise können die Positionierglieder derart gelenkig miteinander verbunden sein, dass sie aufeinander oder ineinander klappbar sind. Auf diese Weise wird es möglich, dass die Vorsatzeinrichtung im zusammengeklappten Zustand der Positionierglieder nur einen geringen Plaztzbedarf hat. Ein solch zusammengeklappter Zustand wird insbesondere dann gewählt, wenn das wenigstens eine Linsenelement nicht benötigt wird und sich somit in einer Art Parkposition befindet. In diesem Fall behindert die Vorsatzeinrichtung den Bediener einer optischen Beobachtungseinrichtung, an der die Vorsatzeinrichtung befestigt ist, nicht.

Gemäß einem bevorzugten Merkmal der Erfindung kann alternativ oder zusätzlich vorgesehen sein, dass die Positioniereinrichtung zwei Positionierglieder aufweist, wobei ein erstes Positionierglied zwei voneinander beanstandete Schenkel aufweist, die einen Aufnahmeraum begrenzen und wobei das andere, zweite Positionierglied über das Gelenk derart mit dem ersten Positionierglied verbunden ist, dass es in dessen Aufnahmeraum hineinklappbar ist. In einem solchen Fall kann das Gelenk bevorzugt als ein wie oben beschriebenes Drehgelenk ausgebildet sein.

Wie weiter oben beschrieben ist, ist die Erfindung nicht auf eine bestimmte Anzahl von Linsenelementen, oder aber bestimmte Typen von Linsenelementen beschränkt. Nachfolgend werden diesbezüglich einige vorteilhafte, jedoch nicht ausschließliche Beispiele erläutert.

Wenn die Vorsatzeinrichtung beispielsweise zum Einsatz an einem Operationsmikroskop für die Ophthalmologie zum Beobachten eines zu operierenden Auges kommt, benötigt der Operateur bei Operationen an der Retina oder am Glaskörper Ansichten, bei denen er das Zentrum der Retina bis hin zu deren Peripherienereichen gut sehen kann. In einem solchen Fall sind unterschiedliche Typen von Linsenelementen erforderlich. Beispielsweise ist ein Linsenelement mit einer geringen Dioptrienzahl von 30D bis 60D ideal für eine große Vergrößerung und eine hohe Auflösung. Ein Linsenelement mit hoher Dioptrien zwischen 90D und 120D ist beispielsweise ideal für eine gute Weitwinkelbetrachtung.

Grundsätzlich ist es für die vorliegende Erfindung ausreichend, wenn an dem Halteelement nur ein einziges Linsenelement vorgesehen ist. Wie vorstehend beschrieben ist, existieren aber auch Anwendungsfälle, in denen zwei oder mehr Linsenelemente vorteilhaft sind.

Vorteilhaft kann daher vorgesehen sein, dass an dem Halteelement zwei oder mehr Linsenelemente, vorzugsweise lösbar, angeordnet sind. Dabei kann beispielsweise vorgesehen sein, dass jeweils ein Linsenelement mit geringer Dioptrien und ein Linsenelement mit hoher Dioptrien, so wie oben geschildert, verwendet wird. Somit können über die Vorsatzeinrichtung zwei Linsenelemente unterschiedlichen Typs bereitgestellt werden, beispielsweise ein Linsenelement für eine hohe Auflösung und ein Linsenelement für eine gute Weitwinkelbetrachtung. Während einer Operation kann der Operateur das jeweils gewünschte Linsenelement wählen, beispielsweise durch Rotation des Halteelements. Dies ist insbesondere bei solchen Operationen von Vorteil, bei denen häufig zwischen verschiedenen Linsenelementen umgeschaltet werden muss.

Gemäß einem bevorzugten Merkmal der Erfindung ist vorgesehen, dass wenigstens ein Linsenelement als Ophthalmoskopierlupe ausgebildet ist.

Ophthalmoskopierlupen bestehen in der Regel aus einer einfachen, oft asphärischen Linse. Sie haben in der Regel keinen Kontakt mit dem Patientenauge - der Abstand zum Patientenauge ist abhängig von den Lupeneigenschaften und liegt vorzugsweise bei etwa 3 bis 25 mm -, und lassen sich einfacher reinigen/sterilisieren. Sie werden bevorzugt bei chirurgischen Eingriffen eingesetzt, und zwar wegen ihrer "non-contact"-Eigenschaft und ihrer Sterilisierbarkeit.

Die Ophthalmoskopierlupen bestehen vorteilhaft aus der Linse und einer Lupenfassung mit einer Koppelstelle/Aufnahme für Lupenhalter - zur Befestigung an einem Halteelement. Die Eigenschaften der Linse werden vorteilhaft für die Erfordernisse bei der Hinterabschnittschirurgie angepasst und unterscheiden sich im Wesentlichen in ihrer Brechkraft. Abhängig von der Brechkraft der Linse muss jede Ophthalmoskopierlupe in einer definierten Position zum Patientenauge positioniert werden, um den Hinterabschnitt des Patientenauges optimal, das heißt formatfüllend, abzubilden. Das Halteelement befindet sich im eingeschwenkten Zustand in einem definierten Abstand zur Beobachtungseinrichtung beziehungsweise zum Patientenauge. Die Abstandsdifferenzen der unterschiedlichen Ophthalmoskopierlupen werden beispielsweise über unterschiedlich starke Kröpfungen der Lupenfassung realisiert.

Wenn die Linsenelemente aus dem Strahlengang des Operationsmikroskops herausgeschwenkt sind, können auf besonders einfache Weise auch so genannte Kontaktgläser zum Einsatz kommen. Kontaktgläser bestehen meist aus mehreren Linsen / Linsengruppen. Sie haben direkten Kontakt mit dem Patientenauge, lassen sich bezüglich ihrer optischen Abbildungseigenschaften gezielter optimieren und einsetzen, sind aber meist nicht / bedingt oder nur sehr aufwendig sterilisierbar. Kontaktgläser werden bevorzugt bei Diagnostik oder Kontrolle eingesetzt.

Vorteilhaft kann vorgesehen sein, dass das wenigstens eine Linsenelement fest an dem Halteelement abgeordnet ist. In anderer Ausgestaltung ist auch denkbar, dass wenigstens ein Linsenelement positionsveränderlich, beispielsweise verschiebbar, an dem Halteelement angeordnet ist. Auch ist es möglich, dass das Halteelement eine Art Linsenaufnahme bildet, an der lösbar das wenigstens eine Linsenelement über einen Linsenhalter befestigt ist. Dies kann beispielsweise realisiert werden, indem der Linsenhalter in das Halteelement einsteckbar ist.

Die Erfindung ist nicht auf bestimmte Ausgestaltungsformen für das Halteelement beschränkt. Nachfolgend werden einige bevorzugte Ausführungsbeispiele für Halteelemente beschrieben.

Vorteilhaft ist vorgesehen, dass das Halteelement als Haltearm ausgebildet ist und/oder dass das Halteelement drehbar an der Positioniereinrichtung angeordnet ist.

Beispielsweise kann das Halteelement als Haltearm ausgebildet sein. In diesem Fall ist das wenigstens eine Linsenelement vorteilhaft am Ende des Haltearms angeordnet. Wenn zwei oder mehr Linsenelemente zum Einsatz kommen, kann der Haltearm zwei oder mehr in Bezug auf den Befestigungspunkt des Haltearms an der Positioniereinrichtung vom Befestigungspunkt abragende Armbereiche aufweisen, wobei jeweils ein Linsenelement am Ende eines solchen Armbereichs angeordnet ist.

In anderer Ausgestaltung kann vorgesehen sein, dass das Halteelement in Form einer Linsenaufnahme ausgebildet ist. An dieser Linsenaufnahme werden die Linsenelemente befestigt. Dazu sind entsprechende Linsenhalter vorgesehen, an denen die Linsenelemente, vorteilhaft mittels einer Linsenfassung, befestigt werden beziehungsweise gehalten sind. Über die Linsenhalter werden die Linsenelemente dann an der Linsenaufnahme befestigt, vorzugsweise lösbar, etwa mittels einer Steckverbindung.

Im einfachsten Fall kann der Haltearm oder der wenigstens eine Linsenhalter eine gerade Kontur aufweisen. Es sind jedoch auch Haltearmkonturen oder Linsenhalterkonturen vorteilhaft, bei denen der Haltearm beziehungsweise die Armbereiche des Haltearms beziehungsweise der Linsenhalter einen zumindest bereichsweise winkeligen und/oder kurvigen und/oder gekröpften Verlauf haben. Unter einem gekröpften Verlauf soll dabei insbesondere auch ein doppelwinklig abgebogener Verlauf verstanden werden. Insbesondere in den letztgenannten Fällen kann durch einen derartigen nicht geraden Verlauf erreicht werden, dass sich solche Linsenelemente, die gerade nicht benötigt werden, eng an die optische Beobachtungseinrichtung anschmiegen können, so dass sie aus dem Arbeitsfeld der optischen Beobachtungseinrichtung nicht herausragen und auch im Arbeitsfeld selbst nicht störend wirken, so dass der Bediener nicht gestört wird. Weist der Haltearm einen wie vorstehend beschriebenen nicht geraden Verlauf auf, so ist dieser Verlauf vorteilhaft an die Kontur und Geometrie der optischen Beobachtungseinrichtung, für die die Vorsatzeinrichtung zum Einsatz kommen soll, angepasst. Die Armbereiche können allerdings auch als die weiter oben beschriebenen Lupenhalter ausgebildet sein, die dann mit einem als Aufnahme ausgebildeten Halteelement verbunden werden.

Das Halteelement, beispielsweise in Form eines Haltearms oder einer Linsenaufnahme, stellt die Aufnahme für die Linsenelemente dar und dient zur Halterung/Aufnahme der Linsenelemente, beispielsweise der Ophthalmoskopierlupen, während des Einsatzes. Bei Verwendung des Halteelements, das eine Art Linsenelementwechsler darstellt, enthält dieses mindestens zwei Aufnahmestellen. In das Halteelement können die Linsenelemente dann beispielsweise mit entsprechender Fassung ein-/ausgesteckt werden. Bei der Gestaltung des Halteelements ist eine einfache Reinigung- und Sterilisierbarkeit gewährleistet. Durch eine zusätzliche Koppelstelle können die Linsenelemente für Sterilisation und dergleichen vom Halteelement entfernt werden, beispielsweise aus dessen Halterung entnommen werden, zum Beispiel, wenn Linsenelemente mit anderen Reinigungs-/Sterilisationsverfahren wiederaufbereitet werden. Dadurch kann das Halteelement, beispielsweise ein Wechsler mit verschiedenen Linsenelementpaarungen, konfiguriert werden, etwa für den schnellen und unkomplizierten Einsatz während eines chirurgischen Eingriffs.

Vorzugsweise ist deshalb vorgesehen dass das wenigstens eine Linsenelement lösbar an dem Halteelement angeordnet ist.

Die unterschiedlichen linsenelementspezifischen Abstände zur Beobachtungsvorrichtung, beziehungsweise zum Patientenauge, werden vorteilhaft über linsenelementspezifische Fassungen, vorteilhaft mit unterschiedlicher Kröpfung, realisiert.

Vorteilhaft kann vorgesehen sein, dass das Halteelement drehbar an der Positioniereinrichtung angeordnet ist. Auf diese Weise lässt sich das Linsenelement bequem in die gewünschte Arbeitsposition verdrehen. Weist das Halteelement zwei oder mehr Linsenelemente auf, kann auf diese Weise bequem dasjenige Linsenelement in die Arbeitsposition gedreht werden, das gerade benötigt wird. Vorteilhaft kann das Halteelement auch in solch eine Position gedreht werden oder drehbar sein, in der sich kein Linsenelement in der Arbeitsposition, beispielsweise einem bestimmten Strahlengang, befindet. Eine solche Position kann als Neutralposition oder Parkposition bezeichnet werden. In einer solchen Position ist es dann einfach und bequem möglich, mit den weiter oben beschriebenen Kontaktgläsern zu arbeiten.

Wenn das Halteelement in einer solchen Weise drehbar an der Positioniereinrichtung angeordnet ist, kann man dieses als eine Art Revolvereinrichtung oder Bestandteil einer solchen Revolvereinheit bezeichnen. Eine derartige Revolvereinrichtung wird weiter unten in größerem Detail beschrieben, so dass diesbezüglich im Hinblick auf eine solche vorteilhafte Ausgestaltung der Vorsatzeinrichtung auch vollinhaltlich auf die entsprechenden Ausführungen zur Revolvereinrichtung Bezug genommen und verwiesen wird.

Wenn die Vorsatzeinrichtung beispielsweise für ein Operationsmikroskop der Ophthalmologie eingesetzt und als Fundusabbildungssystem verwendet wird, kann beispielsweise eine derartige Parkposition eingestellt werden, in der die Vorsatzeinrichtung an das Mikroskop angeklappt ist und wobei das - vorzugsweise winkelförmige - Halteelement für die Linsenelemente, bei denen es sich beispielsweise um Ophthalmoskopierlupen handeln kann, geeignet verdreht wird, so das sich keines der Linsenelemente im Strahlengang befindet. Dies ermöglicht einem Operateur auch den Einsatz von so genannten Kontaktgläsern bei der Operation eines Patientenauges. Hierbei handelt es sich um Linsenelemente die direkt auf das Patientenauge gelegt werden, und die nicht in einer Halterung aufgenommen sind, welche am Operationsmikroskop befestigt ist.

Wenn die Vorsatzeinrichtung im Zusammenhang mit einem Operationsmikroskop, beispielsweise einem Operationsmikroskop der Ophthalmologie, eingesetzt wird, kann durch Einsatz der Vorsatzeinrichtung die erforderliche Operationszeit verkürzt werden. Der Operateur kann einfach und schnell das erforderliche Linsenelement finden und - vorzugsweise durch Rotation des Halteelements- einsetzen. Ein einziges System kann nunmehr alle unterschiedlichen Operationsarten und Operationsschritte abdecken. Dies reduziert zusätzlich auch die Operationskosten, beispielsweise hinsichtlich Anzahl der zu verwendenden Einrichtungen, hinsichtlich des zu verwendenden Materials, hinsichtlich der durchzuführenden Reinigungen und Sterilisierungsvorgänge, und dergleichen.

Der verhältnismäßig einfache Aufbau der Vorsatzeinrichtung (ohne optische Bauelemente, ohne motorische oder manuelle Stelleinheiten oder aufwendige Getriebe, und dergleichen) ist einfach zu reinigen, zu desinfizieren und zu sterilisieren. Somit ist die gesamte Vorsatzeinrichtung schnell und einfach für den Einsatz unter sterilen Bedingungen zu verwenden.

Insbesondere kann eine Revolvereinrichtung zur drehbaren Anordnung an einer wie vorstehend beschriebenen erfindungsgemäßen Vorsatzeinrichtung vorgesehen sein, so dass alles bezüglich der Revolvereinrichtung Gesagte auch im Zusammenhang mit der erfindungsgemäßen Vorsatzeinrichtung gilt. Vorteilhaft ist in diesem Zusammenhang eine Vorsatzeinrichtung, bestehend aus einer Fokussiereinrichtung, einer Abdeckkappe, einer daran angeordneten Halteeinrichtung und einer entsprechenden Revolvereinrichtung.

Die Revolvereinrichtung weist ein um eine Drehachse drehbares Halteelement auf, das in der wie vorstehend beschriebenen Weise ausgebildet sein kann, an dem die wenigstens zwei Linsenelemente angeordnet sind.

Die gesamte Revolvereinrichtung stellt dann eine Art Linsenelementwechsler dar, wobei sich die Linsenelemente an dem Halteelement befinden und das Halteelemente drehbar gelagert ist, so das die Linsenelementen, ähnlich wie bei der Trommel eines Revolvers, weiter gedreht werden können. Dabei ist insbesondere vorgesehen, dass die einzelnen Linsenelemente jeweils in diskrete Positionen gedreht werden können, beispielsweise eine Parkposition oder eine Arbeitsposition. Natürlich ist auch denkbar, dass die Linsenelemente um bis zu 360 Grad gedreht werden können.

Vorteilhaft kann vorgesehen sein, dass das Halteelement einer solchen Revolvereinrichtung, aber auch das weiter oben beschriebene Halteelement allgemein, als Linsenaufnahme ausgebildet ist, dass die wenigstens zwei Linsenelemente jeweils an einem Linsenhalter angeordnet sind und über diesen, insbesondere lösbar, an der Linsenaufnahme angeordnet sind. Vorteilhaft kann vorgesehen sein, dass wenigstens ein Linsenhalter einen zumindest bereichsweise kurvigen Verlauf oder winkeligen Verlauf oder gekröpften Verlauf aufweist.

Vorteilhaft ist weiterhin, wenn die Drehachse der Revolvereinrichtung zu einer optischen Achse der Beobachtungseinrichtung, an der die Revolvereinrichtung befestigt ist, vorzugsweise mittels einer geeigneten Vorsatzeinrichtung, einen geneigten Verlauf hat.

Durch eine derartige Revolvereinrichtung lassen sich verschiedene Linsenelemente, beispielsweise Ophthalmoskopierlupen, kurzfristig, wechselseitig in den Strahlengang einer Optik, auch während einer Operation, auch mehrmals und auch unter sterilen Bedingungen, einschwenken oder über eine entsprechende Zwischenstellung sämtliche Linsenelemente, beispielsweise Ophthalmoskopierlupen, aus dem Strahlengang ausschwenken. Die Revolvereinrichtung ist über eine entsprechende Ausgestaltung des Halteelements so gestaltet, dass die jeweilige(n) Ophthalmoskopierlupe(n), welche nicht benötigt wird/werden, den Bauraum unterhalb der Beobachtungsvorrichtung möglichst so gering wie möglich stören. Dies wird insbesondere erreicht durch entsprechende winklige Anordnung der Drehgelenke und eine Kröpfung der Linsenhalter.

Die Verwendung einer solchen Revolvereinrichtung, die dann die Funktion eines Ophthalmoskopierlupen-Wechslers hat, ermöglicht das wechselseitige, nicht gleichzeitige Einschwenken verschiedener Ophthalmoskopierlupen sowie das Ausschwenken der (aller) Ophthalmoskopierlupen aus dem Strahlengang der Beobachtungsvorrichtung bei eingeschwenkter Fokussieroptik, beispielsweise bei Verwendung von Kontaktgläsern. Die Revolvereinrichtung und die Ophthalmoskopierlupen, beispielsweise deren Fassung, sind dabei so gestaltet, dass die Lupen, abhängig von ihrer Brechkraft, in unterschiedlichen, definierten Abständen zur Beobachtungsvorrichtung beziehungsweise zu dem Patientenauge positioniert werden können.

Gemäß einem weiteren Aspekt der Erfindung wird ein Operationsmikroskop bereitgestellt, das erfindungsgemäß dadurch gekennzeichnet ist, dass es zumindest eine wie vorstehend beschriebene, erfindungsgemäße Vorsatzeinrichtung aufweist. Zur Vermeidung von Wiederholungen wird deshalb zunächst auf die vorstehenden Ausführungen zur erfindungsgemäßen Vorsatzeinrichtung vollinhaltlich Bezug genommen und verwiesen.

Gemäß noch einem weiteren Aspekt der Erfindung wird ein Operationsmikroskop bereitgestellt, das dadurch gekennzeichnet ist, dass dieses eine wie vorstehend beschriebene erfindungsgemäße Vorsatzeinrichtung aufweist, und dass weiterhin eine Fokussiereinrichtung vorgesehen ist, die in dem Operationsmikroskop integriert ist. Zur Vermeidung von Wiederholungen wird deshalb zunächst auch diesbezüglich auf die vorstehenden Ausführungen zur erfindungsgemäßen Vorsatzeinrichtung vollinhaltlich Bezug genommen und verwiesen. Vorteilhaft kann das Operationsmikroskop auch eine wie vorstehend beschriebene erfindungsgemäße Revolvereinrichtung aufweisen, so dass auch diesbezüglich auf die entsprechenden Ausführungen vollinhaltlich Bezug genommen und verwiesen wird.

Vorzugsweise kann das Operationsmikroskop ein Objektiv aufweisen, wobei die Vorsatzeinrichtung im Umgebungsbereich des Objektivs an dem Operationsmikroskop angeordnet ist. Vorteilhaft kann die Anordnung der Vorsatzeinrichtung im Randbereich des Objektivs erfolgen. Die Anordnung der Vorsatzeinrichtung erfolgt dabei vorteilhaft in einer Weise, dass das Linsenelement in den Strahlengang, der durch das Objektiv hindurch verläuft, eingebracht werden kann.

Vorteilhaft kann vorgesehen sein, dass die Vorsatzeinrichtung schwenkbar und/oder drehbar und/oder linear verschiebbar an dem Operationsmikroskop angeordnet ist.

Die Vorsatzeinrichtung besteht vorteilhaft aus den Grundkomponenten Fokussiereinrichtung, Abdeckkappe, Positioniereinrichtung und Halteeinrichtung mit Halteelement.

Gemäß einem bevorzugten Merkmal der Erfindung kann die Vorsatzeinrichtung lösbar an dem Operationsmikroskop angeordnet sein. Auf diese Weise kann die Vorsatzeinrichtung leicht entfernt werden und beispielsweise einer Reinigung, einer Sterilisierung in einem Autoklaven, oder dergleichen unterzogen werden.

Gemäß einem weiteren bevorzugten Merkmal der Erfindung kann die Fokussiereinrichtung lösbar an dem Operationsmikroskop angeordnet sein. Beispielsweise lässt sich die Fokussiereinrichtung über einen Adapter, etwa eine Bajonett-Schwalbenschwanzführung, dauerhaft, das heißt über die Dauer eines chirurgischen Eingriffes, am Mikroskop befestigen. Auf diese Weise kann sie bei Bedarf über diese Adapter-/Kupplungsstelle zwischen den einzelnen chirurgischen Eingriffen, insbesondere unter unsterilen Bedingungen, auch abgenommen werden. Nach dem Aufsetzen / Aufstecken der Vorsatzeinrichtung beziehungsweise der Fokussiereinrichtung auf den Adapter wird diese über eine geeignete Fixierung, beispielsweise einen Rast- und/oder Klemmmechanismus, gegen ungewolltes Lösen während der Operationen gesichert/fixiert. Die Lösbarkeit der Vorsatzeinrichtung beziehungsweise der Fokussiereinrichtung von dem Operationsmikroskop ist insbesondere dann von Vorteil, wenn dieses über einen längeren Zeitraum beziehungsweise über mehrere Operationen hinweg nicht für Hinterabschnittchirurgie sondern ausschließlich für Vorderabschnittschirurgie verwendet wird, insbesondere hinsichtlich mehr Arbeitsraum, weniger Gewicht, weniger Störkonturen und dergleichen. Die Lösbarkeit der Fokussiereinrichtung von der optischen Beobachtungseinrichtung ermöglicht eine Reduzierung von Störkonturen bei der Vorderabschnittschirurgie.

Die Fokussiereinrichtung an sich ist in der Regel kompliziert aufgebaut und sehr empfindlich. Deshalb wird sie in der Regel auch nicht gereinigt und sterilisiert. Zu diesem Zwecke existiert die wenigstens eine Abdeckkappe, die ihrerseits reinigbar und sterilisierbar ist, und die lösbar mit der Fokussiereinrichtung verbunden ist und diese abdeckt. Der Einsatz der Abdeckkappe mit der daran angeordneten Halteeinrichtung ermöglicht dann ein steriles Arbeiten.

Beispielsweise kann vorgesehen sein, dass die Vorsatzeinrichtung beziehungsweise die Fokussiereinrichtung verschiebbar an dem Operationsmikroskop angeordnet ist. Dies kann beispielsweise über einen Schiebemechanismus, etwa eine Linearführung, erfolgen. Dieser kann vorteilhaft zwei rastende Endpositionen/- lagen aufweisen. So lässt sich die Fokussier-/Reduzieroptik während des chirurgischen Eingriffs temporär, auch mehrmals, in den Strahlengang des Mikroskops einschwenken sowie aus dessen Strahlengang ausschwenken, und zwar derart, dass möglichst minimale Einschränkungen des Arbeitsraumes entstehen, was zu einer bauraumminimierten Ausgestaltung führt. Bei einer solchen Ein-/Ausschwenkbewegung wird die am verschiebbaren Bereich der Fokussiereinrichtung befestigte Abdeckhaube mitbewegt. Ein Ausschwenken ist insbesondere für Arbeiten am Vorderabschnitt sinnvoll / erforderlich, insbesondere zur Vermeidung ungewünschter Reflexe und dergleichen aufgrund der zum Teil komplizierten Beleuchtung am Mikroskop. Darüber hinaus wird der Arbeitsraum unmittelbar unter dem Hauptobjektiv vom Mikroskop freigegeben, was zu einer Reduzierung von Störkonturen, zu mehr Bewegungsfreiheit und dergleichen führt.

Ein weiteres bevorzugtes Merkmal der vorliegenden Erfindung sieht vor, dass eine wie vorstehend beschriebene erfindungsgemäße Vorsatzeinrichtung und/oder ein wie vorstehend beschriebenes erfindungsgemäßes Operationsmikroskop vorteilhaft für die indirekte kontaktlose Ophthalmologie oder die indirekte kontaktbehaftete Ophthalmologie verwendet werden kann/können.

Das Operationsmikroskop kann auch ein System zur Strahlvertauschung und Bildumkehr enthalten, das sich vorteilhaft im Binokulartubus befindet.

Die Erfindung wird nun anhand von Ausführungsbeispielen unter Bezugnahme auf die beiliegenden Zeichnungen näher erläutert. Es zeigen
- Figur 1: eine Explosionsansicht einer erfindungsgemäßen optischen Beobachtungseinrichtung in Form eines Operationsmikroskops;
- Figur 2: eine seitliche Ansicht der optischen Beobachtungseinrichtung mit angesetzter Fokussiereinrichtung;
- Figur 3: die in Figur 2 gezeigte Darstellung, zu Beginn des Ansatzes der Fokussiereinrichtung;
- Figur 4: die in Figur 2 gezeigte Darstellung, bei der sich die Fokussiereinrichtung in einer ersten Verschiebeposition befindet;
- Figur 5: die in Figur 2 gezeigte Darstellung, bei der sich die Fokussiereinrichtung in einer zweiten Verschiebeposition befindet;
- Figur 6: eine perspektivische Ansicht einer optischen Bebachtungseinrichtung mit erfindungsgemäßer Vorsatzeinrichtung, wobei sich diese im ausgeklappten Zustand befindet;
- Figur 7: den in Figur 6 dargestellten Teilausschnitt A in starker Vergrößerung;
- Figur 8: eine seitliche Darstellung einer optischen Beobachtungseinrichtung, kurz vor der Befestigung der Abdeckkappe an der Fokussiereinrichtung;
- Figur 9: die in Figur 8 gezeigte Darstellung nach vollendeter Befestigung der Abdeckkappe an der Fokussiereinrichtung;
- Figur 10: eine Möglichkeit zum Entriegeln der Abdeckkappe zur Lösung derselben von der Fokussiereinrichtung;

- Figur 11: eine Übersicht über die Funktionalitäten der Vorsatzeinrichtung;
- Figur 12: eine Seitenansicht der in Figur 6 dargestellten optischen Beobachtungseinrichtung;
- Figur 13: eine perspektivische Ansicht einer optischen Bebachtungseinrichtung mit erfindungsgemäßer Vorsatzeinrichtung, wobei diese von einem zuvor ausgeklappten Zustand in einen zusammengeklappten Zustand verbracht wird;
- Figur 14: eine Seitenansicht der in Figur 13 dargestellten optischen Beobachtungseinrichtung;
- Figur 15: eine perspektivische Ansicht einer optischen Bebachtungseinrichtung mit erfindungsgemäßer Vorsatzeinrichtung, wobei sich diese in einem zusammengeklappten Zustand befindet;
- Figur 16: eine Seitenansicht der in Figur 15 dargestellten optischen Beobachtungseinrichtung;
- Figur 17: eine Ansicht von unten auf die in den Figuren 15 und 16 dargestellte optische Beobachtungseinrichtung mit zusammengeklappter Vorsatzeinrichtung;
- Figur 18: eine Ansicht gemäß Figur 17, bei der sich die zusammengeklappte Vorsatzeinrichtung in einer Parkposition befindet;
- Figur 19: eine perspektivische Ansicht auf die in Figur 18 dargestellte optische Beobachtungseinrichtung, wobei sich diese in einem zusammengeklappten Zustand und in einer Parkposition befindet;
- Figur 20: eine Detailansicht eines Haltelements zur Aufnahme der Linsenelemente;
- Figur 21: mehrere Detailansichten zu verschiedenartig ausgestalteten Linsenelementen;
- Figur 22: eine erste Arbeitsposition der optischen Beobachtungseinrichtung in Bezug zu einem zu operierenden Patienten;
- Figur 23: eine zweite Arbeitsposition der optischen Beobachtungseinrichtung in Bezug zu einem zu operierenden Patienten;
- Figur 24: eine dritte Arbeitsposition der optischen Beobachtungseinrichtung in Bezug zu einem zu operierenden Patienten; und

- Figur 25: eine vierte Arbeitsposition der optischen Beobachtungseinrichtung in Bezug zu einem zu operierenden Patienten.

In den Figuren ist eine an sich bekannte optische Beobachtungseinrichtung 10 in Form eines Operationsmikroskops für die Ophthalmologie dargestellt. Bei dem Operationsmikroskop 10 soll es sich um ein Ophthalmoskopiemikroskop handeln. Derartige Mikroskope sind dem Fachmann an sich bekannt, so dass auf deren Grundaufbau im vorliegenden Fall nicht weiter eingegangen wird.

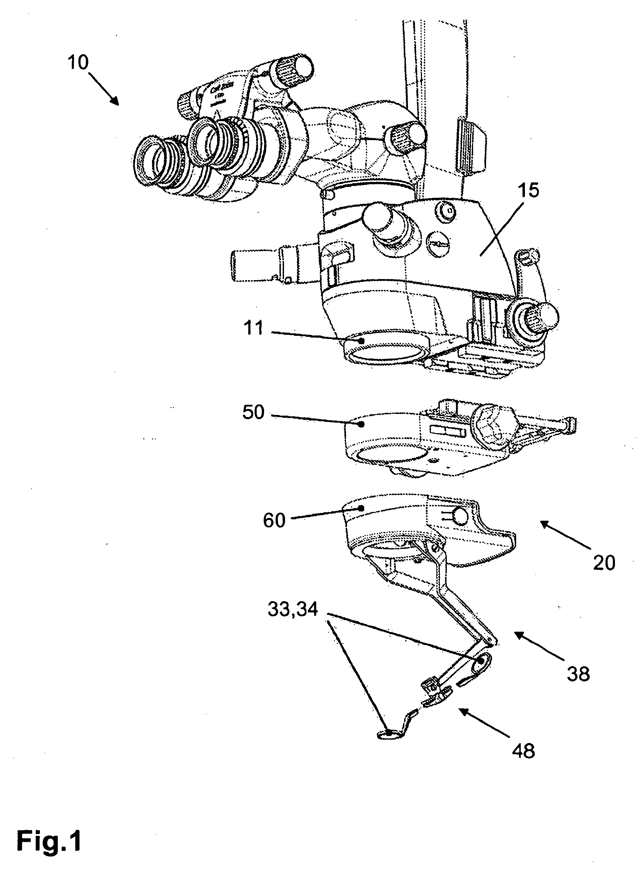

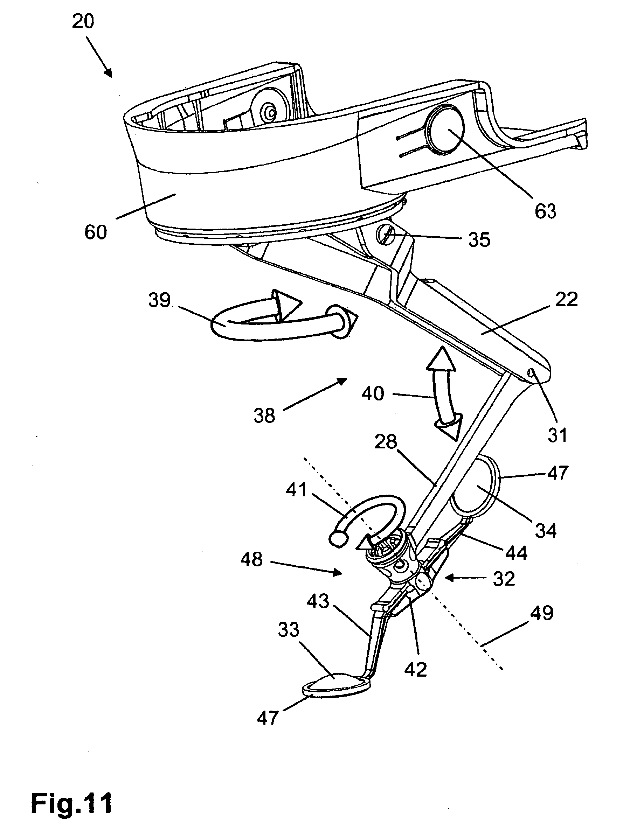

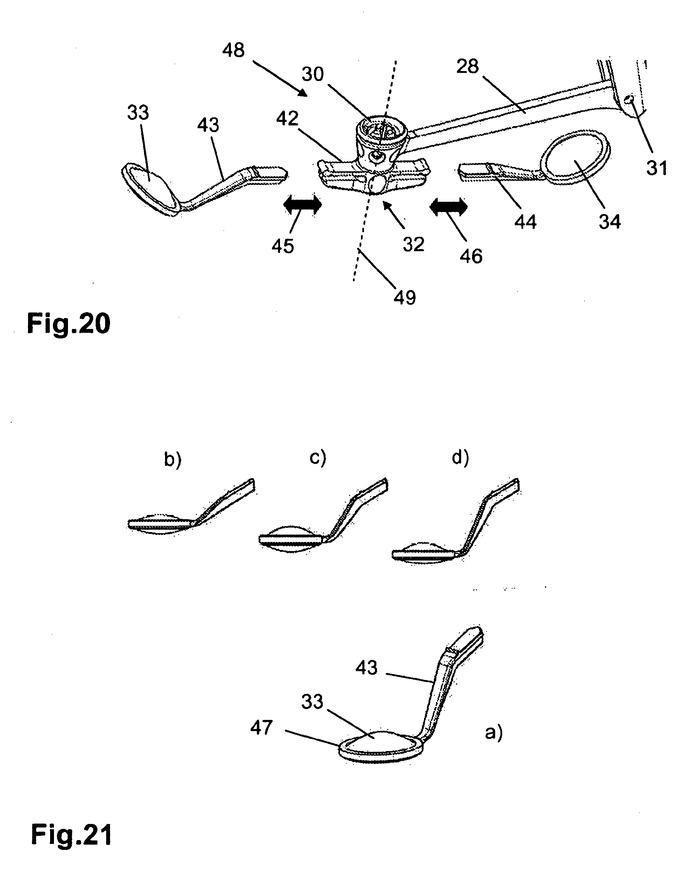

Wie zunächst in Figur 1 verdeutlicht ist, besteht das Mikroskop 10 aus einem Mikroskopkörper 15, welcher ein Objektiv 11 aufweist. Hierbei soll es sich um das Hauptobjektiv des Mikroskops 10 handeln. An der Unterseite des Mikroskopkörpers 15 wird eine Fokussiereinrichtung 50 angeordnet, die weiter unten noch näher spezifiziert wird. An der Fokussiereinrichtung 50, die einen Bestandteil einer Vorsatzeinrichtung 20 darstellt, wird eine Abdeckkappe 60 befestigt. An der Abdeckkappe 60 wiederum ist eine Halteeinrichtung 38 befestigt, durch welche Linsenelemente 33, 34 gehalten werden. Die Linsenelemente 33, 34 sind über eine Revolvereinrichtung 48 drehbar an der Halteeinrichtung 38 befestigt. Die Revolvereinrichtung 48 ist im Zusammenhang mit den Figuren 11 und 20 weiter unten näher erläutert.

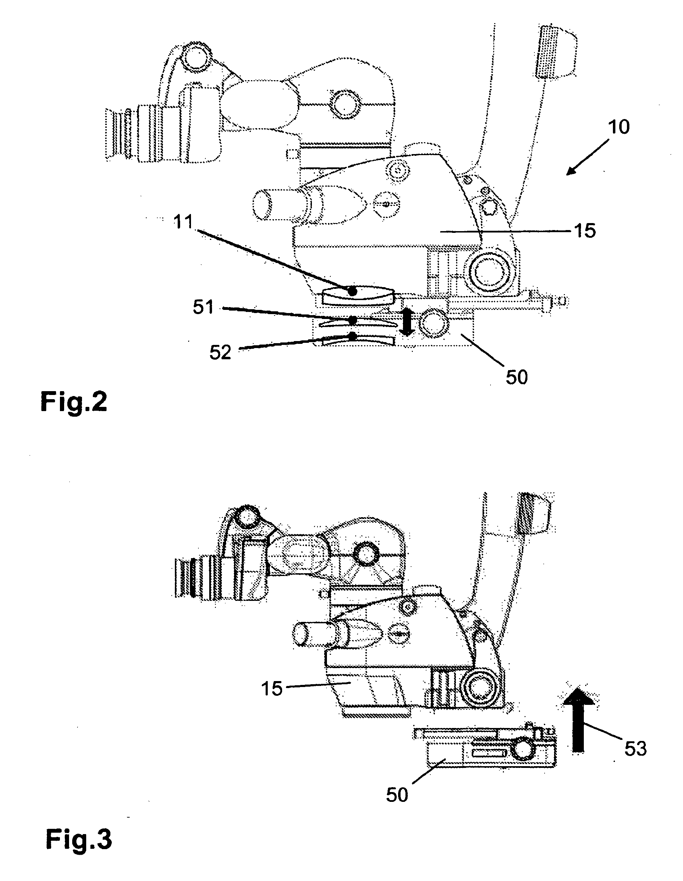

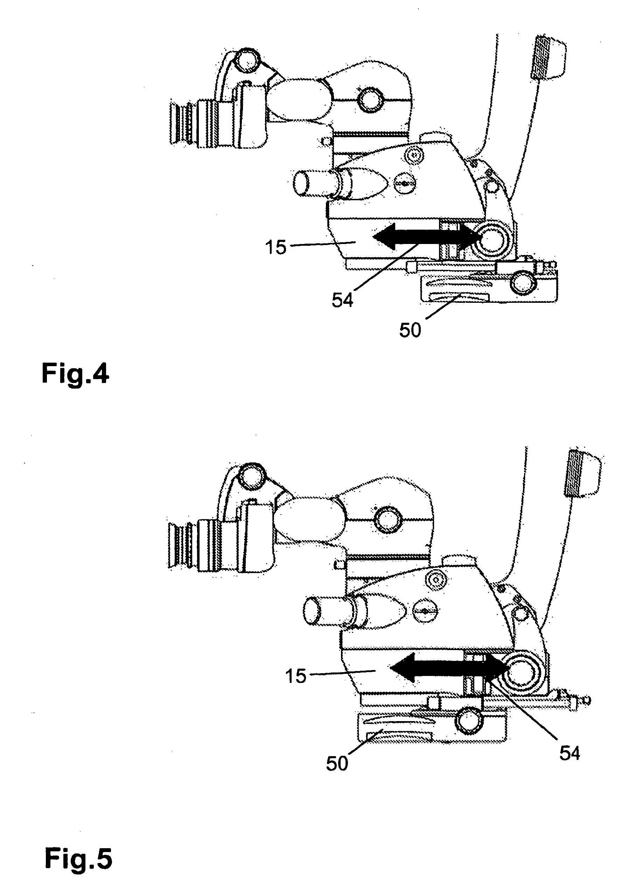

Die Befestigung sowie die Positionierung hinsichtlich eines Ein- und Ausschwenkens der Fokussiereinrichtung 50 an dem Mikroskopkörper 15 wird nachfolgend anhand der Figuren 2 bis 5 beschrieben. Dort ist zunächst dargestellt (Figur 2), dass die Fokussiereinrichtung 50 in Form einer Fokussieroptik, beispielsweise einer Reduzieroptik, ausgebildet ist. Die Fokussiereinrichtung 50 weist zwei optische Bauelemente in Form eines verschiebbaren Positivgliedes 51 und eines positionsfesten Negativgliedes 52 auf. Die optischen Bauelemente 51, 52 liegen mit dem Hauptobjektiv 11 des Mikroskops 10 auf einer optischen Achse. Die optischen Bauelemente 51, 52 der Fokussiereinrichtung 50 dienen zur Fokussierung des Mikroskops 10 auf das Zwischenbild der Linsenelemente 33, 34 (Figur 1). In Figur 3 ist dargestellt, wie die Fokussiereinrichtung 50 zur Befestigung an dem Mikroskop zunächst von unten gegen den Mikroskopkörper 15 angebracht wird. Dies ist durch den Pfeil 53 gekennzeichnet. Nach erfolgter Befestigung kann über einen Schiebemechanismus, eine so genannte Linearführung, der zwei rastende Endpositionen/Endlagen aufweist, erreicht werden, dass sich die Fokussiereinrichtung 50 während eines chirurgischen Eingriffs temporär, auch mehrmals, in den Strahlengang des Mikroskops 10 einschwenken sowie aus diesem ausschwenken lässt. Diese Verschiebung ist in den Figuren 4 und 5 durch den Pfeil 54 gekennzeichnet, wobei in Figur 4 die Fokussiereinrichtung 50 in ausgeschwenktem Zustand, und in Figur 5 die Fokussiereinrichtung 50 in eingeschwenktem Zustand dargestellt ist.

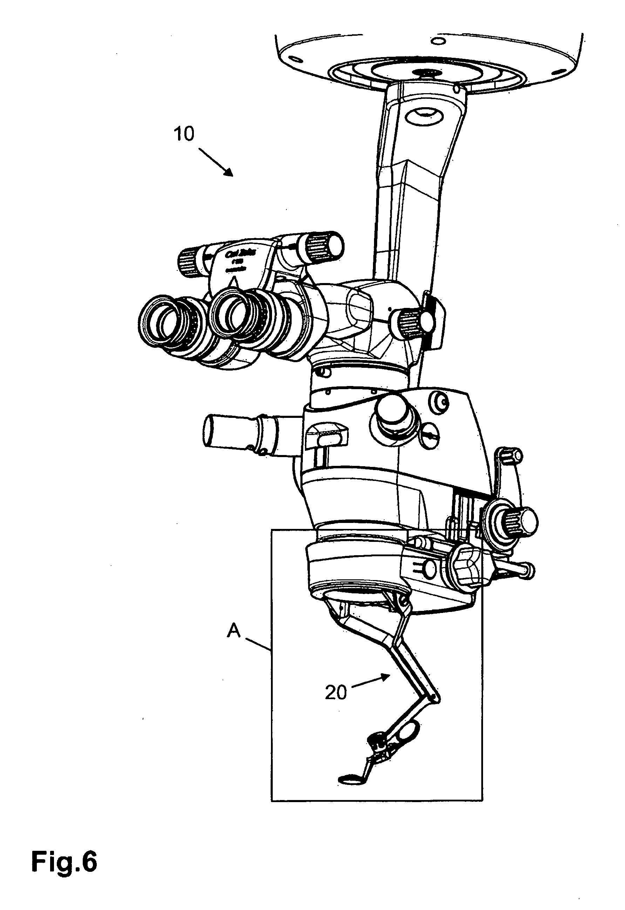

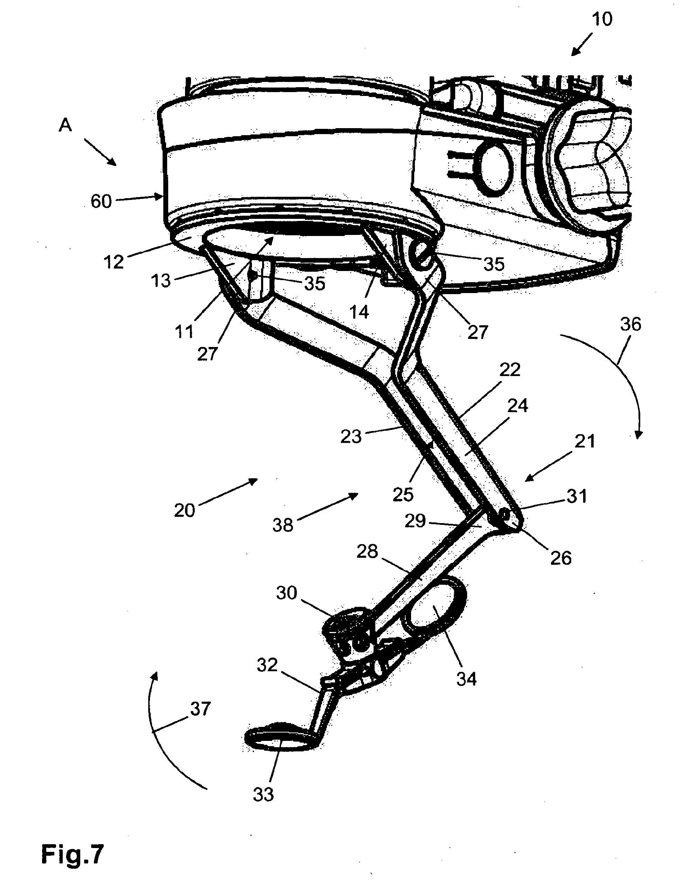

Das Operationsmikroskop 10 verfügt über eine besonders ausgestaltete, erfindungsgemäße Vorsatzeinrichtung 20. Diese erfindungsgemäße Vorsatzeinrichtung 20 ist in Figur 6 als Ausschnitt A gekennzeichnet. Der Ausschnitt A ist in Figur 7 vergrößert dargestellt. Der Aufbau und die Funktionsweise der erfindungsgemäßen Vorsatzeinrichtung 20 werden nachfolgend zunächst im Zusammenhang mit Figur 7 erläutert.

Die Vorsatzeinrichtung 20 soll als Vorsatz für ein Objektiv 11 des Operationsmikroskops 10 dienen. Dazu ist die Vorsatzeinrichtung 20 in einem Bereich des Operationsmikroskops, welcher zum Objektbereich weist, am Operationsmikroskop 10 angeschlossen.

Die Vorsatzeinrichtung 20 verfügt zunächst über zwei Linsenelemente 33, 34, in Form von Ophthalmoskopierlupen. Diese sind über ein Halteelement 32 an einer Positioniereinrichtung 21 angeordnet. Halteelement 32 und Positioniereinrichtung 21 sind Bestandteil einer Halteeinrichtung 38.

Die Positioniereinrichtung 21 stellt ein wesentliches Merkmal der Vorsatzeinrichtung 20 dar. Die Positioniereinrichtung 21 besteht generell aus zwei Positioniergliedern 22, 28, die über ein Gelenk 31, beispielsweise ein Drehgelenk, miteinander verbunden sind.

Im vorliegenden Beispiel ist zunächst ein erstes Positionierglied 22 vorgesehen, welches zwei voneinander beabstandete Schenkel 23, 24 aufweist, die einen Aufnahmeraum 25 begrenzen. An einem Ende 26 des ersten Positionierglieds 22 ist dieses über das Gelenk 31 mit dem zweiten Positionierglied 28 verbunden.

Das zweite Ende 27 des ersten Positionierglieds 22 dient zur Befestigung der Halteeinrichtung 38 an einer Abdeckkappe 60 der Vorsatzeinrichtung 20. Die Abdeckkappe 60 dient dazu, die Fokussiereinrichtung 50 (in Figur 7 nicht dargestellt) steril abzudecken. Die Abdeckkappe 60 ist vorteilhaft lösbar an der Fokussiereinrichtung 50 angeordnet. Auf diese Weise kann die Abdeckkappe 50 samt der Halteeinrichtung 38 vom Mikroskop 10 abgenommen und nach Beendigung der Operation bequem gereinigt, desinfiziert und sterilisiert werden.

Die Befestigung der Halteeinrichtung 38 an der Abdeckkappe 60 erfolgt über eine geeignete Befestigungseinrichtung 35 mit Drehgelenk, beispielsweise eine entsprechend ausgebildete Bolzen- oder Schraubverbindung. Dazu wirkt die Befestigungseinrichtung 35 mit korrespondierenden Befestigungsschenkeln 13, 14, die -in einem Befestigungsbereich 12 an der Abdeckkappe 60 ausgebildet sind, zusammen. Über diese Befestigung ist die Halteeinrichtung 38 mit dem ersten Positionierglied 22 in einer ersten Schwenkrichtung 36 schwenkbar an der Abdeckkappe 60 der Vorsatzeinrichtung 20 angeordnet. Dabei kann vorgesehen sein, dass die Vorsatzeinrichtung 20 lösbar am Operationsmikroskop 10 angeordnet ist. So kann diese zu Reinigungs- und Sterilisierungszwecken leicht abgenommen und beispielsweise in einen Autoklaven verbracht werden.

Das zweite Positionierglied 28 ist stabförmig ausgebildet und an seinem einen Ende 29 über das Gelenk 31 mit dem ersten Positionierglied 22 verbunden. Das Gelenk 31 ist dabei in solch einer Weise ausgebildet, dass die beiden Positionierglieder 22, 28 zusammengeklappt werden können. Dies geschieht in solch einer Weise, dass das zweite Positionierglied 28 im zusammengeklappten Zustand innerhalb des Aufnahmeraums 25 des ersten Positionierglieds 22 zu liegen kommt.

Am anderen Ende 30 des zweiten Positionierglieds 28 ist das Halteelement 32 drehbar angeordnet. Das Halteelement 32 ist dabei als Haltearm ausgebildet, wobei der Haltearm 32 zwei Armbereiche aufweist, die jeweils von der Mittelposition, an der der Haltearm 32 mit dem Ende 30 des Positionierglieds 28 verbunden ist, anragen. Jeweils an den äußeren Enden des Haltearms 32 sind die Linsenelemente 33, 34 vorgesehen.

Der Haltearm 32, und mit ihm die Linsenelemente 33, 34 sind über das zweite Positionierglied 28 und das Gelenk 31 in einer zur ersten Schwenkrichtung 36 abweichenden zweiten Schwenkrichtung 37 schwenkbar.

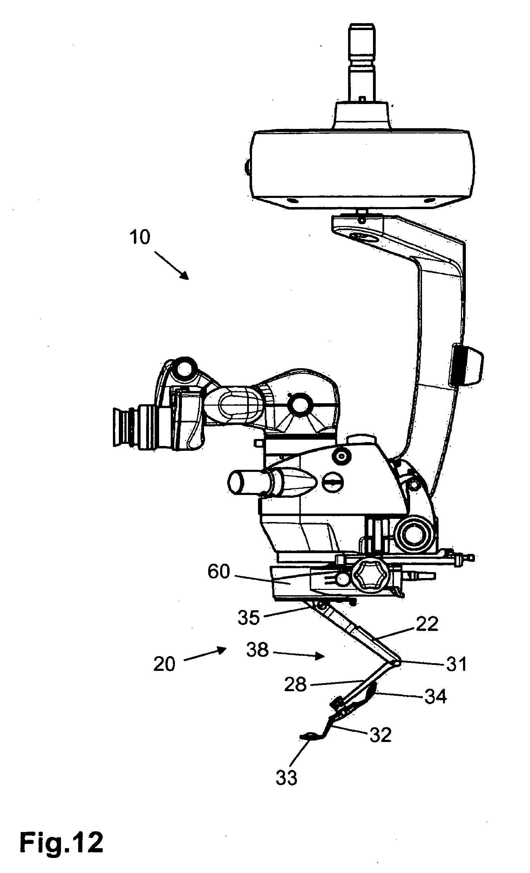

In den Figuren 6, 7 und 12 ist ein erster Zustand dargestellt, in dem die Vorsatzeinrichtung 20 vollständig ausgeklappt ist. Diese Ausklappposition kann beispielsweise aufgrund der Gewichtskraft eingenommen werden. Wenn etwa eine entsprechende Fixierung gelöst wird, strebt die Vorsatzeinrichtung allein aufgrund der Gewichtskraft in die ausgeklappte Position. Durch entsprechende Drehung des Haltearms 32 ist es dem Operateur möglich, wahlweise das erste Linsenelement 33 oder das zweite Linsenelement 34 in einen Strahlengang, der durch das Objektiv 11 hindurch verläuft, einzubringen, beziehungsweise beide Linsenelemente aus dem Strahlengang herauszuschwenken.

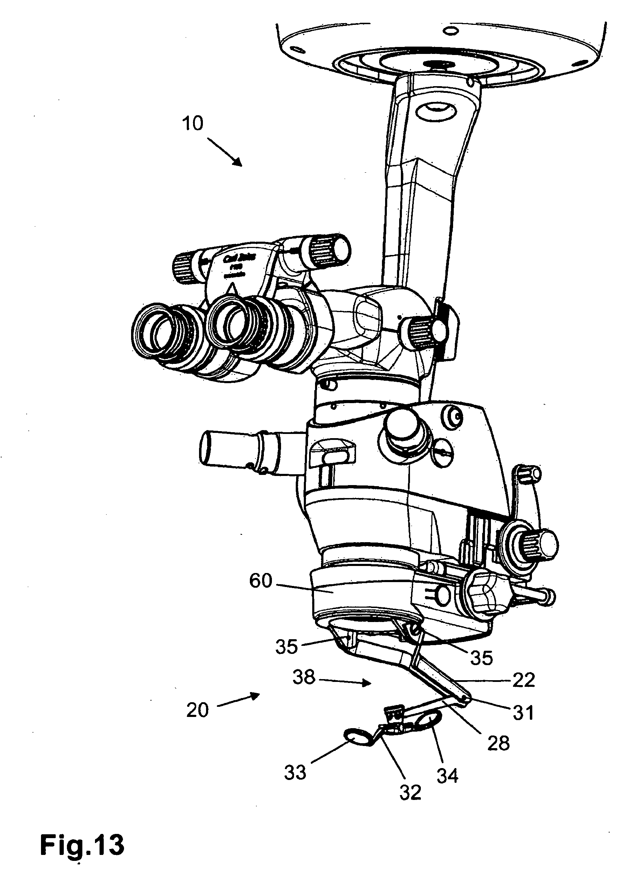

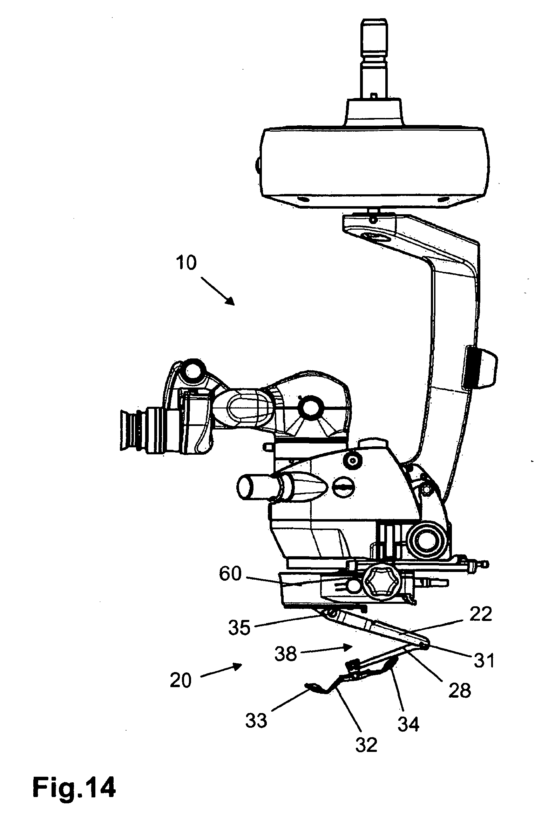

In den Figuren 13 und 14 ist ein zweiter Zustand dargestellt, in dem die Vorsatzeinrichtung 20 ein Stück weit zusammengeklappt ist. Hierbei soll es sich im Ausführungsbeispiel nicht um eine Arbeitsposition handeln. Vielmehr ist in den genannten Figuren eine Zwischenposition dargestellt, die die Beweglichkeit der Positioniereinrichtung 21 auf Basis eines Schwerenmechanismus verdeutlicht.

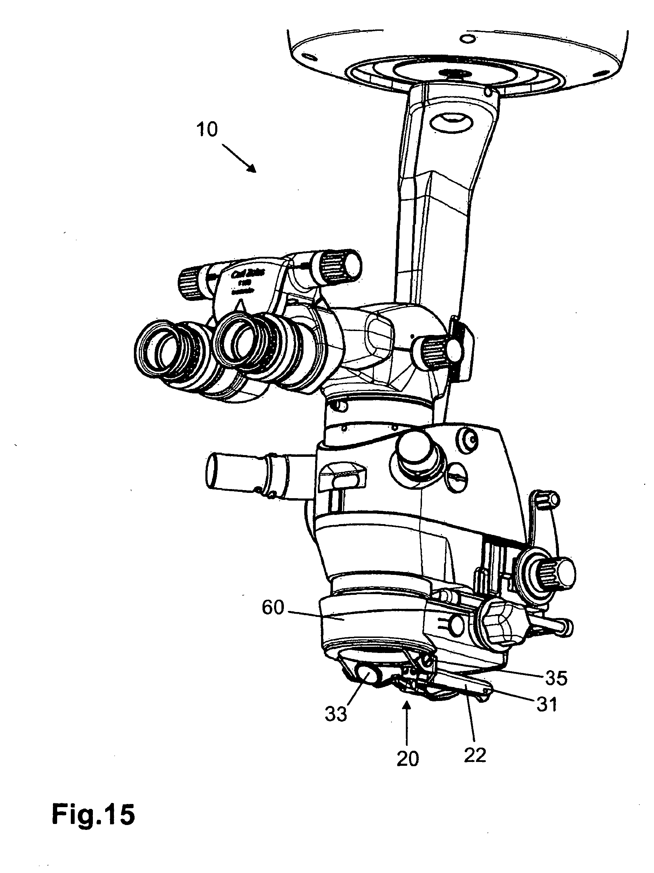

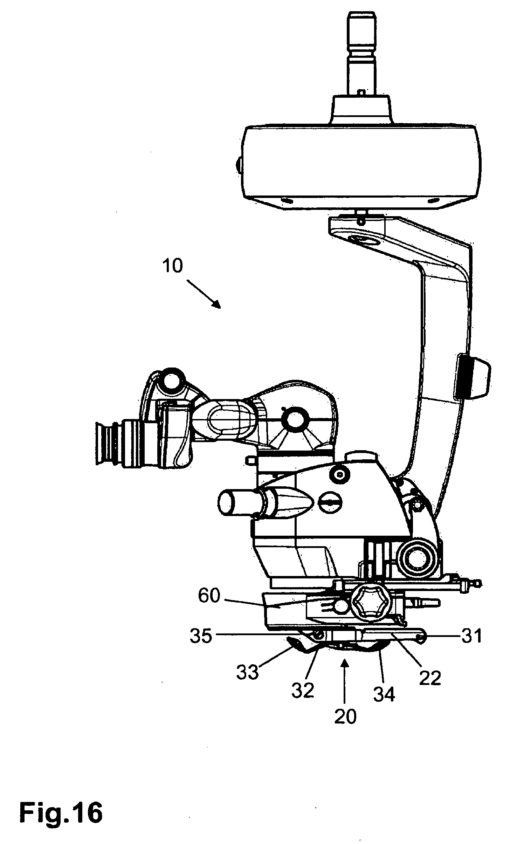

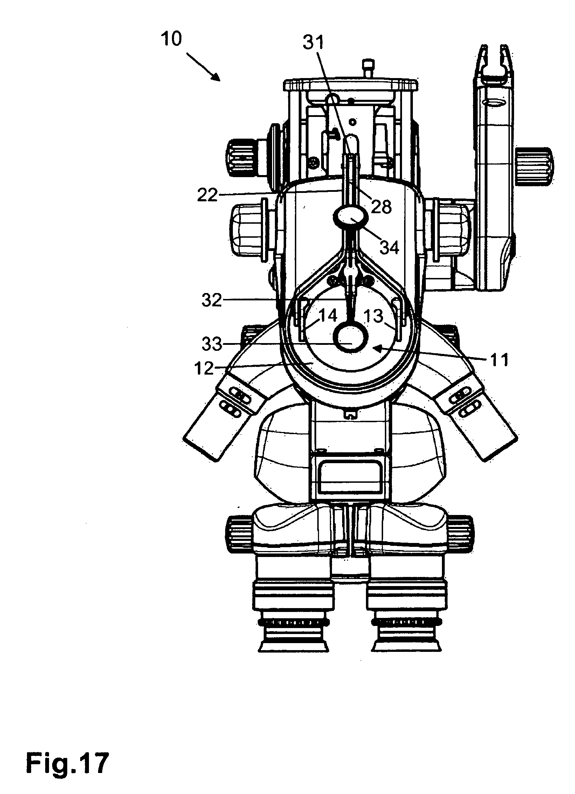

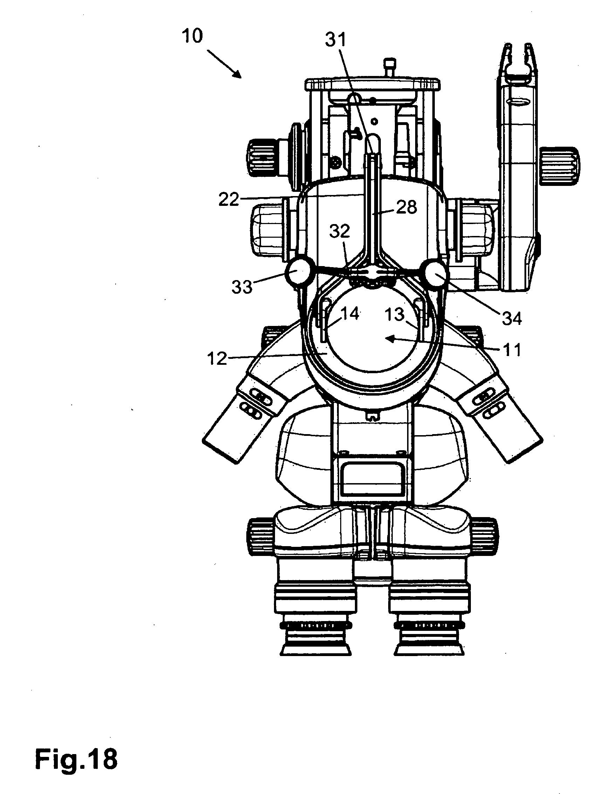

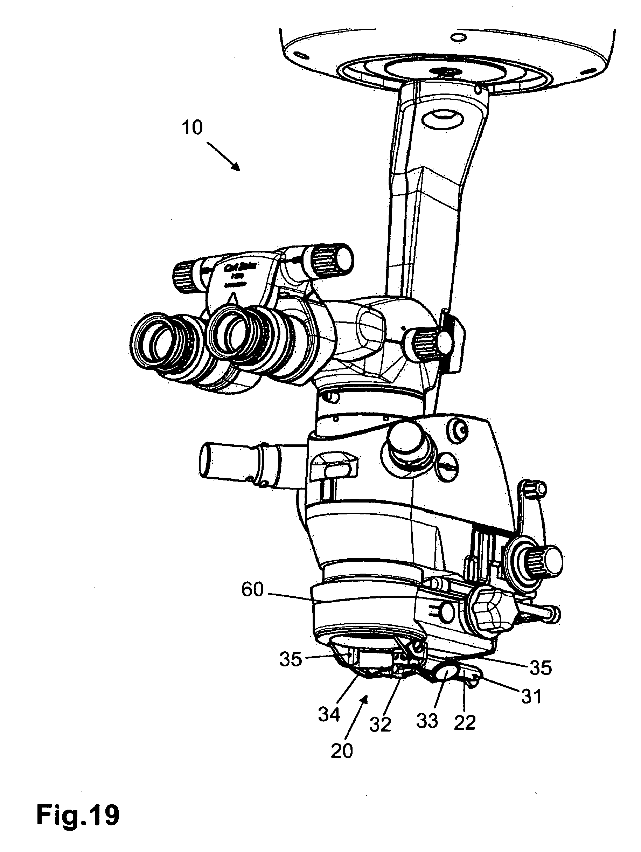

In den Figuren 15 bis 19 schließlich ist die Vorsatzeinrichtung 20 im zusammengeklappten Zustand dargestellt. Zunächst wird deutlich, dass durch einen zumindest zum Teil winkeligen Verlauf des Haltearms 32 erreicht werden kann, dass die Linsenelemente 33, 34, zumindest aber dasjenige Linsenelement, welches gerade nicht benötigt wird, sehr eng am Operationsmikroskop 10 anliegen/anliegt, so dass sie/es im Betrieb für den Operateur nicht störend sind/ist. In den Figuren 15, 16 und 17 ist eine Situation dargestellt, in der sich das Linsenelement 33 im Strahlengang befindet. In den Figuren 18 und 19 ist indes eine Situation dargestellt, in der sich die Linsenelemente 33, 34 in einer Art Parkposition befinden, in der sich also kein Linsenelement im Strahlengang befindet. Eine solche Parkposition ist beispielsweise dann vorteilhaft, wenn ein Kontaktglas zum Einsatz kommen soll, das direkt auf das Patientenauge aufgelegt wird, so dass die Linsenelemente 33, 34 nicht benötigt werden. In einem solchen Fall ist dann aber vorteilhaft immer noch die Fokussiereinrichtung 50 aktiv, um die Arbeit mit einem Kontaktglas zu ermöglichen.

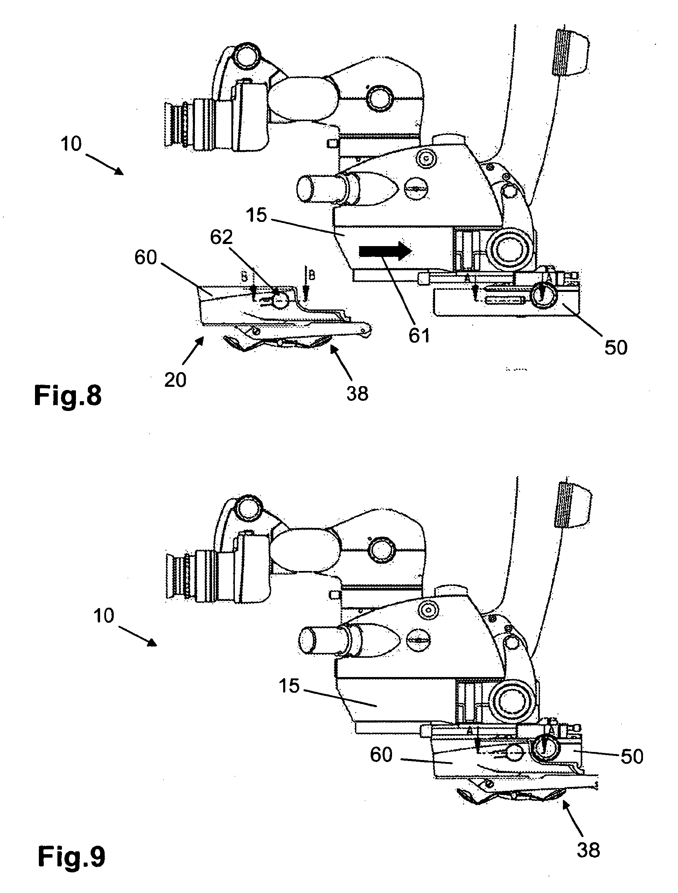

In den Figuren 8 bis 10 ist dargestellt, wie die Vorsatzeinrichtung 20 über die Abdeckkappe 60, an der sich die Halteeinrichtung 38 befindet, an dem Mikroskopkörper 15 des Mikroskops 10 befestigt wird.

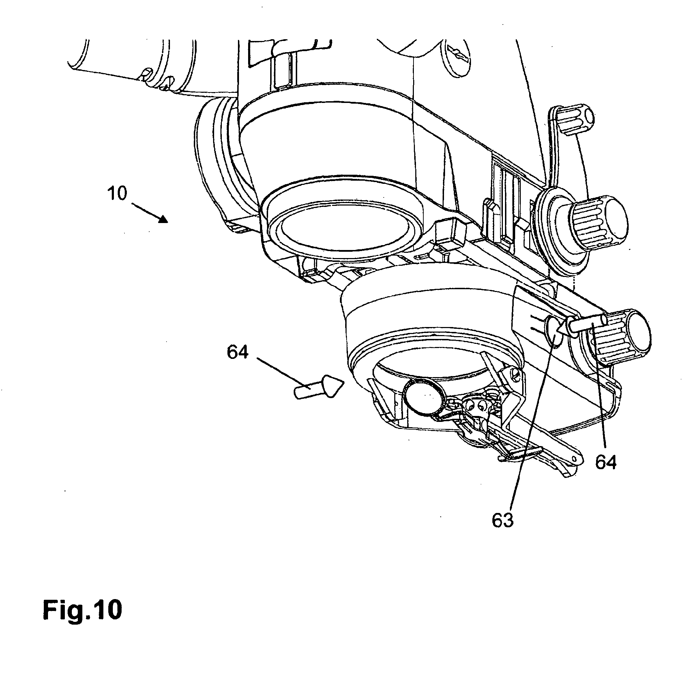

Die sterile Abdeckkappe 60 lässt sich temporär auf den verschiebbaren Bereich der Fokussiereinrichtung 50 - auch unter sterilen Bedingungen während der Operation (bei Bedarf auch mehrmals) - aufstecken. Die Aufsteckrichtung ist dabei durch Pfeil 61 gekennzeichnet. Wie in Figur 8 zu sehen ist, wird die Abdeckkappe 60 von vorne auf die Fokussiereinrichtung 50 aufgesteckt. Beim Aufstecken verrastet die Kappe 60 so, dass die Abdeckkappe 60 zur Fokussiereinrichtung 50 ausgerichtet und gegen ungewolltes Lösen während der Operation gesichert ist. Die Fixierung erfolgt mittels einer Fixiereinrichtung 62, bei der Rastnasen einen Hinterschnitt hintergreifen können. Die Rastnasen können über eine Federeinrichtung in dieser Fixierposition gehalten werden. Die Abdeckkappe 60 in aufgestecktem Zustand ist in Figur 9 dargestellt. Das Entrasten beziehungsweise Lösen der Abdeckkappe 60 kann über zwei Bedienelemente / Entriegelungsknöpfe 63 an der Abdeckkappe 60 erfolgen. Durch Drücken der Knöpfe 63 in Entriegelungsrichtung 64 (Figur 10) wird die Rastnase aus der Hintergreifung herausgehoben, wodurch die Abdeckkappe 60 von der Fixiereinrichtung 50 gelöst und von dieser abgezogen werden kann.

Figur 11 zeigt eine Übersicht über die verschiedenen Funktionalitäten der Vorsatzeinrichtung 20. Die Vorsatzeinrichtung 20 ist so ausgelegt, dass die Abdeckkappe 60 zunächst lösbar an der Fokussiereinrichtung 50 befestigt ist. Eine Lösung kann durch Drücken der Entriegelungsknöpfe 63 herbeigeführt werden. Die Halteeinrichtung 38 ist über das erste Positionierglied 22 und die Befestigungseinrichtung 35 an der Abdeckkappe 60 befestigt. Dabei kann vorgesehen sein, dass die Halteinrichtung 38 drehbar beziehungsweise rotierbar an der Abdeckkappe 60 angeordnet ist, was durch den Rotationspfeil 39 dargestellt ist. Weiterhin können die beiden Positionierglieder 22, 28 über das Drehgelenk 31 zusammengeklappt und auseinandergeklappt werden, was durch den Pfeil 40 verdeutlicht ist, der den Öffnungswinkel zwischen den beiden Positioniergliedern 22, 28 verdeutlicht. Die Linsenelemente 33, 34 sind über den Haltearm 32 drehbar beziehungsweise rotierbar am zweiten Positionierglied 28 angeordnet, was durch den Pfeil 41 gekennzeichnet ist.

In Figur 11 ist auch die weiter oben schon genannte Revolvereinrichtung 48 in größerem Detail dargestellt. Diese besteht zunächst aus einem Halteelement 32, das in Form einer Linsenaufnahme 42 ausgebildet ist. Diese Linsenaufnahme 42 ist um eine Drehachse 49 drehbar am Ende des zweiten Positioniergliedes 28 angeordnet. Dabei ist vorgesehen, dass die Drehachse 49 in Bezug zur optischen Achse des Mikroskops geneigt ist. Die Revolvereinrichtung 48 ermöglicht eine Drehung der Linsenelemente 33, 34 um die Drehachse 41. Dazu sind die Linsenelemente 33, 34,die in entsprechenden Linsenfassungen 47 gehalten sind, über diese an gekröpften Linsenhaltern 43, 44 befestigt. Die Linsenhalter 43, 44 wiederum sind, vorzugsweise lösbar, an der Linsenaufnahme 42 befestigt.

Ein weiteres verdeutlichendes Beispiel für das Halteelement 32 ist im Zusammenhang mit Figur 20 näher beschrieben. Das Halteelement 32 verfügt zunächst über eine Linsenaufnahme 42, die drehbar beziehungsweise rotierbar am freien Ende 30 des zweiten Positionierglieds 28 gelagert ist. Das Positionierglied 28 wiederum ist über das Drehgelenk 31 mit dem ersten Positionierglied verbunden. In die Linsenaufnahme 42 können Linsenhalter 43, 44 eingesteckt werden, an denen sich wiederum die Linsenelemente 33, 34 befinden. Die Steckrichtungen sind dabei durch die Pfeile 45 und 46 gekennzeichnet. Diese Steckverbindung hat den Vorteil, dass die Linsenelemente auf einfache Weise von der Linsenaufnahme 42 abgenommen werden können, beispielsweise zu Reinigungszwecken und dergleichen.

In der Figur 21 sind verschiedene Ausgestaltungen von Linsenelementen dargestellt, bei denen es sich um Ophthalmoskopierlupen handeln soll. Das eigentliche Linsenelement 33 befindet sich in einer Linsenfassung 47 und ist über diese am Linsenhalter 43 befestigt (Figur 21 a). In Figur 21 b ist eine Ophthalmoskopierlupe mit geringer Brechkraft, in Figur 21 c ist eine Ophthalmoskopierlupe mit mittlerer Brechkraft, und in Figur 21 d ist eine Ophthalmoskopierlupe mit hoher Brechkraft dargstellt.

In Figur 21 sind Linsenhalter 43 mit unterschiedlich starken Kröpfungen dargestellt. Diese unterschiedlich starken Kröpfungen liegen in den unterschiedlichen optimalen Arbeitsabständen der Ophthalmoskopierlupen zum Patientenauge begründet.

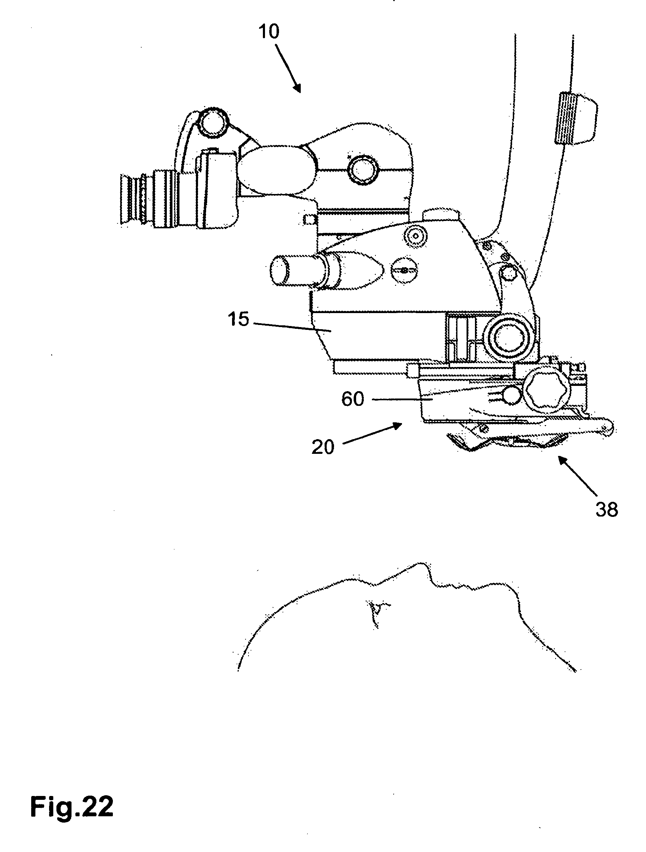

In den Figuren 22 bis 25 schließlich sind verschiedene typische Arbeitssituationen dargestellt.

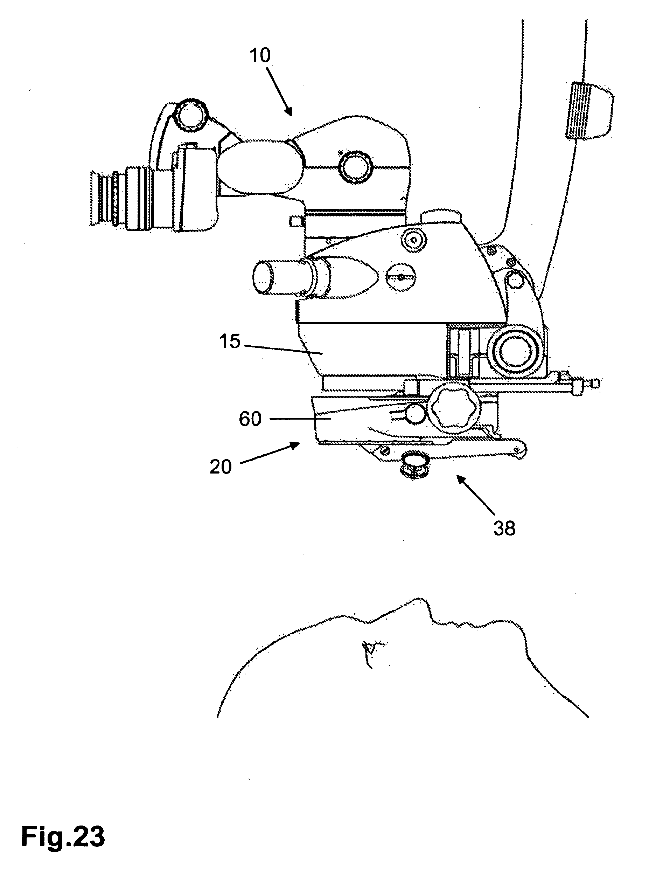

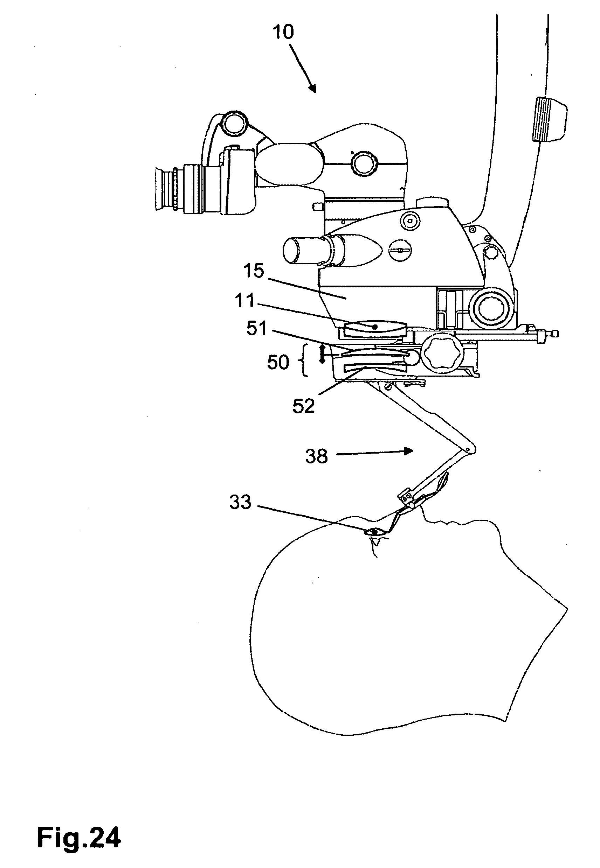

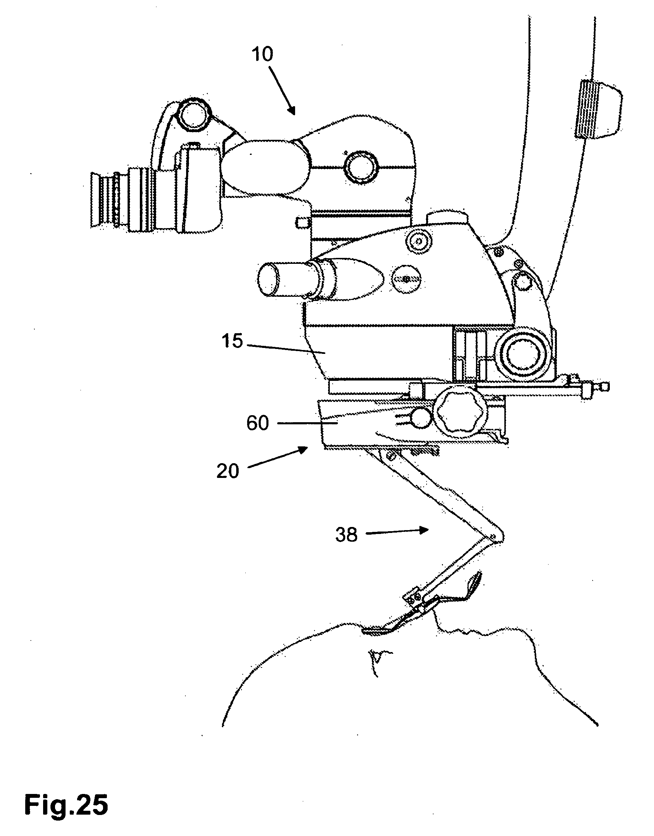

In Figur 22 ist eine Situation dargestellt, die für das Arbeiten am vorderen Augenabschnitt geeignet ist. Es erfolgt ein Fokussieren des Operationsmikroskops bei ausgeschwenkter Vorsatzeinrichtung und aus dem Strahlengang geschalteter Fokussieroptik auf die Ebene Iris des Patientenauges. Figur 23 zeigt die Situation für das Arbeiten mit indirekten Kontaktgläsern, wobei ein Kontaktglas allerdings nicht explizit dargestellt ist. Die Fokussiereinrichtung ist eingeschwenkt, der Lupenwechsler befindet sich in einer Parkposition. Figur 24 zeigt die prinzipielle Anordnung der optischen Bauelemente der Vorsatzeinrichtung bei der indirekten kontaktlosen Ophthalmoskopie mit einem Operationsmikroskop. Die Ophthalmoskopierlupe wurde eingeschwenkt und zwar koaxial zur optischen Achse und auf einen festen, von der Brechkraft der Ophthalmoskopierlupe abhängigen, definierten Abstand zum Mikroskop und somit auch zum Patientenauge. Der Abstand zum Patientenauge ist so gewählt, dass eine optimale, das heißt eine formatfüllende Abbildung ohne Vignettierung erreicht wird. In der Regel existiert zu diesem Zeitpunkt noch kein scharfes Bild. Bei Bedarf kann zum Wechseln der Lupen das Halteelement bedient, insbesondere gedreht beziehungsweise rotiert, werden. Dies ist im Zusammenhang mit Figur 25 gezeigt. Dort hat ein Wechsel/Einschwenken einer/der anderen/gewünschten Ophthalmoskopierlupe stattgefunden, und zwar koaxial zur optischen Achse und auf einen festen, von der Brechkraft der Ophthalmoskopierlupe abhängigen, definierten Abstand zum Mikroskop und somit auch zum Patientenauge. Der Abstand zum Patientenauge ist dabei so gewählt, dass eine optimale, das heißt formatfüllende Abbildung ohne Vignettierung erreicht wird. In der Regel existiert zu diesem Zeitpunkt noch kein scharfes Bild. Anschließend erfolgt ein Verstellen der Fokussieroptik, um das System auf ein von der Ophthalmoskopierlupe generiertes-Zwischenbild des Augenhintergrunds oder Bereichs des Glaskörpers (x mm über Retina) scharf zu stellen.

Die Lage der Zwischenbildebene ist dabei abhängig vom Typ beziehungsweise der Brechkraft der Ophthalmoskopierlupe, von der Fehlsichtigkeit des zu betrachtenden Auges, vom zu betrachtenden Bereich / Abschnitt des Auges, von Anomalien des Auges, oder vom Abstand der Ophthalmoskopierlupe zum Auge.

Somit ist bei entsprechender Positionierung der Ophthalmoskopierlupe beziehungsweise eines Kontaktglases eine Betrachtung des Augenhintergrundes unter verschiedensten Bedingungen und für unterschiedliche Ophthalmoskopierlupen beziehungsweise Kontaktgläser ohne Positionsänderungen an der Beobachtungsvorrichtung möglich, der Abstand Patientenauge zu Mikroskop bleibt unverändert. Daraus resultiert ein schneller und unkomplizierter Wechsel zwischen Betrachtung des Augenvorder- und -hintergrundes. Darüber hinaus lässt sich ein solches System einfach einrichten und manipulieren. Das Fokussieren und die Manipulation der Pupillenlage erfolgt über zwei voneinander entkoppelte Bewegungen.

Die in den Figuren dargestellte Vorsatzeinrichtung kann auf konstruktiv einfache Weise hergestellt und ebenso einfach am Operationsmikroskop befestigt sowie von diesem gelöst werden. Durch die Verschwenkbarkeit der Positionierglieder um das Gelenk kann die Vorsatzeinrichtung komplett zusammengeklappt werden, so dass sie sehr platzsparend am Operationsmikroskop gehalten werden kann, insbesondere dann, wenn sie nicht benötigt wird. Da auch das erste Positionierglied über die Befestigungseinrichtung schwenkbar am Operationsmikroskop angeordnet ist, kann die Halteeinrichtung mit den Linsenelementen auf einfache Weise in einen Bereich verschwenkt werden, wo diese nicht störend sind. Außerdem kann der erforderliche Bauraum reduziert werden. Die Vorsatzeinrichtung lässt sich auf einfache Weise bedienen und in die verschiedenen Positionen verfahren, ohne dass hierzu besondere Antriebe erforderlich wären.

### Bezugszeichenliste

- 10: Optische Beobachtungseinrichtung (Operationsmikroskop)
- 11: Objektiv
- 12: Befestigungsbereich
- 13: Befestigungsschenkel
- 14: Befestigungsschenkel
- 15: Mikroskopkörper
- 20: Vorsatzeinrichtung
- 21: Positioniereinrichtung
- 22: Positionierglied (erstes Positionierglied)
- 23: Schenkel
- 24: Schenkel
- 25: Aufnahmeraum
- 26: Ende des (ersten) Positionierglieds
- 27: Ende des (ersten) Positionierglieds
- 28: Positionierglied (zweites Positionierglied)
- 29: Ende des (zweiten) Positionierglieds
- 30: Ende des (zweiten) Positionierglieds
- 31: Gelenk (Drehgelenk)
- 32: Halteelement (Haltearm)
- 33: Erstes Linsenelement (Ophthalmoskopierlupe)
- 34: Zweites Linsenelement (Ophthalmoskopierlupe)
- 35: Befestigungseinrichtung
- 36: Erste Schwenkrichtung
- 37: Zweite Schwenkrichtung
- 38: Halteeinrichtung
- 39: Rotationsrichtung
- 40: Klapprichtung, beziehungsweise Öffnungswinkel
- 41: Rotationsrichtung
- 42: Linsenaufnahme
- 43: Linsenhalter (erstes Linsenelement)
- 44: Linsenhalter (zweites Linsenelement)
- 45: Steckrichtung
- 46: Steckrichtung
- 47: Linsenfassung
- 48: Revolvereinrichtung
- 49: Drehachse der Revolvereinrichtung
- 50: Fokussiereinrichtung
- 51: Optisches Bauelement (Positivglied)
- 52: Optisches Bauelement (Negativglied)
- 53: Aufsteckrichtung
- 54: Verschieberichtung
- 60: Abdeckkappe
- 61: Aufsteckrichtung
- 62: Fixiereinrichtung
- 63: Entriegelungsknopf
- 64: Entriegelungsrichtung
- A: Ausschnitt aus der optischen Beobachtungseinrichtung gemäß Figur 6

## Patentansprüche

1. Vorsatzeinrichtung (20) für ein Operationsmikroskop (10) mit einer Fokussiereinrichtung (50) und mit einer Halteeinrichtung (38), aufweisend ein Halteelement (32), an welchem wenigstens ein Linsenelement (33, 34) angeordnet ist, sowie weiter aufweisend eine Positioniereinrichtung (21) zum Positionieren des Halteelements (32) und des daran angeordneten wenigstens einen Linsenelements (33, 34) in Bezug auf die Fokussiereinrichtung (50), wobei das Halteelement (32) an der Positioniereinrichtung (21) angeordnet ist, **dadurch gekennzeichnet, dass** die Vorsatzeinrichtung (20) eine Abdeckkappe (60) für die Fokussiereinrichtung (50) umfasst, an der die Halteeinrichtung (38) über eine Befestigungseinrichtung (35) befestigt ist und dass die wenigstens eine Abdeckkappe (60) mit der Fokussiereinrichtung (50) verbunden ist.

2. Vorsatzeinrichtung nach Anspruch 1, **dadurch gekennzeichnet, dass** die Fokussiereinrichtung (50) wenigstens ein optisches Bauelement (50, 51) zur Fokussierung einer Beobachtungseinrichtung auf das Zwischenbild des wenigstens einen Linsenelements (33, 34) aufweist.

3. Vorsatzeinrichtung nach Anspruch 1 oder 2, **dadurch gekennzeichnet, dass** die Halteeinrichtung (38) lösbar und/oder drehbar und/oder schwenkbar und/oder linear verschiebbar an der Abdeckkappe (60) angeordnet ist.

4. Vorsatzeinrichtung nach einem der Ansprüche 1 bis 3, **dadurch gekennzeichnet, dass** die Positioniereinrichtung (21) wenigstens zwei Positionierglieder (22, 28) aufweist, die über ein Gelenk (31) miteinander verbunden sind.

5. Vorsatzeinrichtung nach Anspruch 4, **dadurch gekennzeichnet, dass** die Positioniereinrichtung (21) ein erstes Positionierglied (22) aufweist, das an seinem einen Ende (26) ein Gelenkelement zur gelenkigen Verbindung mit einem zweiten Positionierglied (28) aufweist, dass am anderen Ende (27) des ersten Positionierglieds (22) die Befestigungseinrichtung (35) vorgesehen ist und/oder dass die Positioniereinrichtung (21) ein zweites Positionierglied (28) aufweist, das an seinem einen Ende (29) ein Gelenkelement zur gelenkigen Verbindung mit einem ersten Positionierelement (22) aufweist, und dass am anderen Ende (30) des zweiten Positionierglieds (28) das Halteelement (32) angeordnet ist.

6. Vorsatzeinrichtung nach Anspruch 4 oder 5, **dadurch gekennzeichnet, dass** die Positionierglieder (22, 28) derart gelenkig miteinander verbunden sind, dass sie aufeinander oder ineinander klappbar sind und/oder dass die Positioniereinrichtung (21) zwei Positionierglieder (22, 28) aufweist, wobei ein erstes Positionierglied (22) zwei voneinander beanstandete Schenkel (23, 24) aufweist, die einen Aufnahmeraum (25) begrenzen und wobei das andere, zweite Positionierglied (28) über das Gelenk (31) derart mit dem ersten Positionierglied (22) verbunden ist, dass es in den Aufnahmeraum (25) des ersten Positionierglieds hineinklappbar ist.

7. Vorsatzeinrichtung nach einem der Ansprüche 1 bis 6, **dadurch gekennzeichnet, dass** an dem Halteelement (32) zwei oder mehr Linsenelemente (33, 34) angeordnet sind.

8. Vorsatzeinrichtung nach einem der Ansprüche 1 bis 7, **dadurch gekennzeichnet, dass** das Halteelement (32) als Haltearm ausgebildet ist und/oder dass das Halteelement (32) drehbar an der Positioniereinrichtung (21) angeordnet ist.

9. Vorsatzeinrichtung nach einem der Ansprüche 1 bis 8, **dadurch gekennzeichnet, dass** diese eine Revolvereinrichtung (48) zur drehbaren Anordnung von wenigstens zwei Linsenelementen (33, 34) an der Vorsatzeinrichtung (20) aufweist und dass die Revolvereinrichtung (48) ein um eine Drehachse (49) drehbares Halteelement (32) aufweist, an dem die wenigstens zwei Linsenelemente (33, 34) angeordnet sind.

10. Vorsatzeinrichtung nach Anspruch 9, **dadurch gekennzeichnet, dass** das Halteelement (32) als Linsenaufnahme (42) ausgebildet ist, dass die wenigstens zwei Linsenelemente (33, 34) jeweils an einem Linsenhalter (43, 44), der insbesondere zumindest bereichsweise einen kurvenförmigen Verlauf, oder winkeligen Verlauf, oder gekröpften Verlauf, aufweist, angeordnet sind und dass die wenigstens zwei Linsenelemente (33, 34) über den Linsenhalter (43, 44), insbesondere lösbar, an der Linsenaufnahme (42) angeordnet sind.

11. Vorsatzeinrichtung nach Anspruch 9 oder 10, **dadurch gekennzeichnet, dass** die Drehachse (49) der Revolvereinrichtung (48) zu einer optischen Achse des Operationsmikroskops (10) einen geneigten Verlauf hat.

12. Operationsmikroskop (10) **dadurch gekennzeichnet, dass** dieses wenigstens eine Vorsatzeinrichtung (20) nach einem der Ansprüche 1 bis 11 aufweist.

13. Operationsmikroskop (10) nach Anspruch 12, **dadurch gekennzeichnet, dass** dieses ein Objektiv (11) aufweist und dass die Vorsatzeinrichtung 20) im Umgebungsbereich (12) des Objektivs (11) an dem Operationsmikroskop (10) angeordnet ist.

14. Operationsmikroskop (10) nach Anspruch 12 oder 13, **dadurch gekennzeichnet, dass** die Vorsatzeinrichtung (20) schwenkbar und/oder drehbar und/oder linear verschiebbar am Operationsmikroskop (10) angeordnet ist.

## Claims

1. A front-lens attachment (20) for a surgical microscope (10), with a focussing device (50) and with a retaining device (38), having a retaining element (32), on which at least one lens element (33, 34) is disposed, as well as further having a positioning device (21) for positioning the retaining element (32) and the at least one lens element (33, 34) disposed thereon relative to focussing device (50), wherein retaining element (32) is disposed on positioning device (21), **characterized in that** the font-lens attachment (20) comprises a covering cap (60) for focussing device (50) to which retaining device (38) is fastened by means of a fastening means (35) and that the at least one covering cap (60) is joined with focussing device (50).

2. The front-lens attachment according to claim 1, further **characterized in that** focussing device (50) has at least one optical component (50, 51) for focussing an observation device onto the intermediate image of the at least one lens element (33, 34).

3. The front-lens attachment according to claim 1 or 2, further **characterized in that** retaining device (38) is disposed on covering cap (60) in a detachable and/or rotatable and/or pivotable and/or linearly movable manner.

4. The front-lens attachment according to one of claims 1 to 3, further **characterized in that** positioning device (21) has at least two positioning components (22, 28), which are joined together via a joint (31).

5. The front-lens attachment according to claim 4, further **characterized in that** positioning device (21) has a first positioning component (22), which has on its one end (26) a joint element for articulated joining with a second positioning component (28), that fastening means (35) is provided on the other end (27) of the first positioning component (22) and/or that positioning device (21) has a second positioning component (28), which has on its one end (29) a joint element for articulated joining with a first positioning component (22) and that the retaining element (32) is disposed on the other end (30) of the second positioning component (28).

6. The front-lens attachment according to claim 4 or 5, further **characterized in that** positioning components (22, 28) are joined together in an articulated manner in such way that they can be folded up or spread apart and/or that positioning device (21) has two positioning components (22, 28), that a first positioning component (22) has two legs (23, 24) distanced from one another, which bound an uptake space (25) and that the other, second positioning component (28) is joined with first positioning component (22) via joint (31) in such a way that it can be folded into the uptake space (25) of the first positioning component.

7. The front-lens attachment according to one of claims 1 to 6, further **characterized in that** two or more lens elements (33, 34) are disposed on retaining element (32).

8. The front-lens attachment according to one of claims 1 to 7, further **characterized in that** retaining element (32) is designed as a retaining arm and/or that retaining element (32) is disposed on positioning device (21) in a rotatable manner.

9. The front-lens attachment according to one of claims 1 to 8, further **characterized in that** it comprises a turret attachment (48) for the rotatable arrangement of at least two lens elements (33, 34) on front-lens attachment (20), and that turret attachment (48) has a retaining element (32) that can rotate around an axis of rotation (49), on which the at least two lens elements (33, 34) are disposed.

10. The front-lens attachment according to claim 9, further **characterized in that** retaining element (32) is designed as a lens uptake (42), that the at least two lens elements (33, 34) are each disposed on a lens holder (43, 44), said lens holder (43, 44) particularly having a curve-shaped course or an angular course, or a bent course, at least in regions, and that the at least two lens elements (33, 34) are disposed on lens uptake (42) via the lens holder (43, 44), particularly in a detachable manner.

11. The front-lens attachment according to claim 9 or 10, further **characterized in that** the axis of rotation (49) of the turret attachment (48) has an inclined course relative to an optical axis of the surgical microscope (10).

12. A surgical microscope (10), **characterized in that** it has at least one front-lens attachment (20) according to one of claims 1 to 11.

13. The surgical microscope (10) according to claim 12, further **characterized in that** it has an objective (11) and that the front-lens attachment (20) is disposed in region (12) surrounding objective (11) on surgical microscope (10).

14. The surgical microscope (10) according to claim 12 or 13, further **characterized in that** front-lens attachment (20) is disposed in a pivotable and/or rotatable and/or linearly movable manner on surgical microscope (10).

## Revendications

1. Dispositif de montage (20) pour un microscope opératoire (10) avec un dispositif de mise au point (50) et avec un dispositif de retenue (38), présentant un élément de retenue (32) sur lequel au moins un élément de lentille (33, 34) est disposé, ainsi que présentant en outre un dispositif de positionnement (21) pour le positionnement de l'élément de retenue (32) et de l'au moins un élément de lentille (33, 34) disposé dessus par rapport au dispositif de mise au point (50), l'élément de retenue (32) étant disposé sur le dispositif de positionnement (21), **caractérisé en ce que** le dispositif de montage (20) comporte un capuchon (60) pour le dispositif de mise au point (50), sur lequel le dispositif de retenue (38) est fixé par le biais d'un dispositif de fixation (35) et **en ce que** l'au moins un capuchon (60) est relié au dispositif de mise au point (50).

2. Dispositif de montage selon la revendication 1, **caractérisé en ce que** le dispositif de mise au point (50) présente au moins un élément structurel optique (50, 51) pour la mise au point d'un dispositif d'observation sur l'image intermédiaire de l'au moins un élément de lentille (33, 34).

3. Dispositif de montage selon la revendication 1 ou 2, **caractérisé en ce que** le dispositif de retenue (38) est disposé de manière détachable et/ou rotative et/ou pivotante et/ou mobile linéairement sur le capuchon (60).

4. Dispositif de montage selon l'une quelconque des revendications 1 à 3, **caractérisé en ce que** le dispositif de positionnement (21) présente au moins deux organes de positionnement (22, 28) qui sont reliés entre eux par une articulation (31).

5. Dispositif de montage selon la revendication 4, **caractérisé en ce que** le dispositif de positionnement (21) présente un premier organe de positionnement (22) qui présente sur son extrémité (26) un élément d'articulation pour la liaison articulée avec un second organe de positionnement (28), **en ce que** le dispositif de fixation (35) est prévu sur l'autre extrémité (27) du premier organe de positionnement (22) et/ou **en ce que** le dispositif de positionnement (21) présente un second organe de positionnement (28) qui présente sur son extrémité (29) un élément d'articulation pour la liaison articulée avec un premier élément de positionnement (22) et **en ce que** l'élément de retenue (32) est disposé sur l'autre extrémité (30) du second organe de positionnement (28).

6. Dispositif de montage selon la revendication 4 ou 5, **caractérisé en ce que** les organes de positionnement (22, 28) sont reliés entre eux de manière articulée de telle manière qu'ils soient rabattables l'un sur l'autre ou l'un dans l'autre et/ou en ce que le dispositif de positionnement (21) présente deux organes de positionnement (22, 28), un premier organe de positionnement (22) présentant deux branches (23, 24) espacées l'une de l'autre qui délimitent un espace de réception (25) et l'autre second organe de positionnement (28) étant relié par l'articulation (31) au premier organe de positionnement (22) de telle manière qu'il puisse être rabattu dans l'espace de réception (25) du premier organe de positionnement.

7. Dispositif de montage selon l'une quelconque des revendications 1 à 6, **caractérisé en ce que** deux éléments de lentille (33, 34) ou plus sont disposés sur l'élément de retenue (32).

8. Dispositif de montage selon l'une quelconque des revendications 1 à 7, **caractérisé en ce que** l'élément de retenue (32) est réalisé comme un bras de retenue et/ou **en ce que** l'élément de retenue (32) est disposé de manière rotative sur le dispositif de positionnement (21).

9. Dispositif de montage selon l'une quelconque des revendications 1 à 8, **caractérisé en ce que** celui-ci présente un dispositif revolver (48) pour la disposition rotative d'au moins deux éléments de lentille (33, 34) sur le dispositif de montage (20) et **en ce que** le dispositif revolver (48) présente un élément de retenue (32) pouvant tourner autour d'un axe de rotation (49), sur lequel les au moins deux éléments de lentille (33, 34) sont disposés.

10. Dispositif de montage selon la revendication 9, **caractérisé en ce que** l'élément de retenue (32) est réalisé comme un logement de lentille (42), **en ce que** les au moins deux éléments de lentille (33, 34) sont disposés respectivement sur un support de lentille (43, 44) qui présente en particulier au moins par endroits un tracé en forme de courbe ou angulaire ou coudé et **en ce que** les au moins deux éléments de lentille (33, 34) sont disposés par le biais du support de lentille (43, 44), en particulier de manière détachable, sur le logement de lentille (42).

11. Dispositif de montage selon la revendication 9 ou 10, **caractérisé en ce que** l'axe de rotation (49) du dispositif revolver (48) présente un tracé incliné par rapport à un axe optique du microscope opératoire (10).

12. Microscope opératoire (10), **caractérisé en ce que** celui-ci présente au moins un dispositif de montage selon l'une quelconque des revendications 1 à 11.

13. Microscope opératoire (10) selon la revendication 12, **caractérisé en ce que** celui-ci présente un objectif (11) et **en ce que** le dispositif de montage (20) est disposé dans la zone environnante (12) de l'objectif (11) sur le microscope opératoire (10).

14. Microscope opératoire (10) selon la revendication 12 ou 13, **caractérisé en ce que** le dispositif de montage (20) est disposé de manière pivotante et/ou rotative et/ou mobile linéairement sur le microscope opératoire (10).
